# EUROPÄISCHE PATENTANMELDUNG

(11) **EP 1 595 957 A1**
(43) Veröffentlichungstag der Anmeldung: **16.11.2005**
(21) Anmeldenummer: 04011247.6
(22) Anmeldetag: 12.05.2004
(51) Int. Cl.: C12Q 1/68

(54) **Phosphoglyceratmutasen und Enzymprodukte**

(71) Anmelder: Eigenbrodt, Erich, 35440 Linden (DE)
(72) Erfinder: Engel, Matthias, 66482 Zweibrücken (DE); Eigenbrodt, Erich, 35440 Linden (DE); Mazurek, Sybille, 35440 Linden (DE); Welter, Cornelius, 66693 Mettlach (DE)

(57) **Zusammenfassung**

Die Erfindung beschreibt ein Verfahren, bei dem Phosphoglyceratmutasen und Bisphosphoglyceratmutasen zur Identifizierung therapeutisch nutzbarer Verbindungen verwendet werden. Weiterhin betrifft die Erfindung Proteine oder Peptide mit Phosphoglyceratmutase-Sequenzen und Bisphosphoglyceratmutase-Sequenzen, hierfür kodierende Nukleinsäuren, Vektoren enthaltend solche Nukleinsäuren, und Zellen, welche mit solchen Vektoren transformiert sind, sowie deren Verwendung für therapeutische Zwecke. Die Erfindung schließt auch ein, anstelle der Erhöhung der Bisphosphoglyceratmutase-Aktivität das Enzymprodukt oder analoge Verbindungen therapeutisch zu verwenden.

SEQUENCE LISTING

<110>  Eigenbrodt, Erich

<120>  Phosphoglyceratmutasen und Enzymprodukte

<130>  EI/EP2004

<160>  4

<170>  PatentIn version 3.1

<210>  1
<211>  8
<212>  PRT
<213>  Artificial

<400>  1
Pro Trp Leu Ile Arg His Gly Glu
1               5

<210>  2
<211>  17
<212>  PRT
<213>  Artificial

<400>  2
Met Arg Gln Ile Lys Ile Trp Phe Pro Asn Arg Arg Met Lys Trp Lys
1               5                   10                  15

Lys

<210>  3
<211>  6
<212>  PRT
<213>  Artificial

<400>  3

His His His His His His
1               5


<210>  4

<211>  31

<212>  PRT

<213>  Artificial



<400>  4

Met Arg Gln Ile Lys Ile Trp Phe Pro Asn Arg Arg Met Lys Trp Lys
1               5                   10                  15


Lys His His His His His His Pro Trp Leu Ile Arg His Gly Glu
            20                  25                  30

## Beschreibung

Krebs ist eine weit verbreitete Erkrankung, welche jedenfalls in schweren Fällen im wesentlichen durch operative Maßnahmen bekämpft wird. Eine Behandlung mit zytotoxischen Substanzen ist meist nur eingeschränkt wirksam, da Tumorzellen oft Resistenzen bilden, wobei transmembrane "Pumpen" die Substanz aus der Tumorzelle hinaustransportieren. Ein anderer Ansatz einer Chemotherapie umfaßt den spezifischen Eingriff in den Stoffwechsel von Tumorzellen, wobei tumortypische Reaktionskaskaden der Energiegewinnung oder der Biosynthese gehemmt werden mit dem Resultat einer Inhibierung der Proliferation und sogar der Apoptose der Tumorzellen. Eine Hauptenergiequelle für maligne Tumorzellen ist die Glykolyse, insbesondere die Metabolisierung von Glucose zu Pyruvat.

Teil des Glykolyse-Enzym-Komplexes ist die Phosphoglyceratmutase(PGM), insbesondere Typ B (EC 5.4.2.1). Um die PGM zu aktivieren, ist eine "primende" Phosphorylierung erforderlich, die durch den Kofaktor 2,3-Bisphosphoglycerat (2,3-BPG)hervorgerufen wird. Die aktive PGM im Glykolyse-Enzym-Komplex erlaubt eine komplette Glucose-Metabolisierung zur Gewinnung von ATP und Synthesebausteinen für die Aminosäuresynthese und damit letztendlich die Proliferation der Tumorzelle.
In gesunden Zellen mit physiologisch intakter Regulation der Energiegewinnung besitzt die PGM keine Schrittmacherfunktion. Dagegen deuten Studien mit Tumorzelllinien und transformierten Zellen darauf hin, dass die PGM dort an der Regulation und Koordination der Glykolyse und der Glutaminolyse (ein weiterer Weg zur Energiegewinnung in Tumorzellen) beteiligt ist (Mazurek et al., *J. Cell Physiol*. 181: 136-146 (1999); Mazurek et al., *Biochem J* 356: 247-256 (2001)).
Aktivierung von PGM kann durch Anwesenheit von 2,3-Bisphosphoglycerat erfolgen. 2,3-Bisphosphoglycerat wird in der Zelle synthetisiert aus dem Glykolyse-Zwischenprodukt 1,3-Bisphosphoglycerat durch die 2,3-Bisphosphoglyceratmutase (BisPGM, E.C. 5.4.2.4).

Aus der Literatur ist bekannt, dass die spezifische Aktivität der BisPGM in einigen Tumoren im Vergleich zum korrespondierenden gesunden Gewebe erniedrigt ist (Durany N, Joseph J, Campo E, et al., *Br. J Cancer* 75: 969-977 (1997); Durany, N., et al., *Br. J Cancer 76,* 1139-1149 (1997); Durany N, Joseph J, Jimenez OM, et al., *Br. J Cancer* 82: 20-27 (2000); Hegde, P., et al., *Cancer Res*. 61: 7792-7797 (2001)). Daraus lässt sich schließen, dass auch das Enzymprodukt 2,3-BPG in Tumoren in reduzierter Quantität vorliegen dürfte. Diese Vermutung wird bestätigt durch die Befunde von Yeoh, *Nature* 285: 108-109 (1980), der nachgewiesen hat, dass in proliferierenden, undifferenzierten Zellen die Gehalte an 2,3-BPG bis unter die Nachweisgrenze des Messverfahrens fallen. Im Gegensatz dazu stiegen die 2,3-BPG-Konzentrationen in zur Differenzierung gebrachten Leukämiezellinien wieder an.

Das Enzym PGM liegt in der Zelle gebunden im sogenannten Glykolyseenzym-Komplex vor, der durch Isolelektrische Fokusierungstechniken isolierbar ist. Der Komplex enthält außer PGM noch mehrere andere Glykolyseenzyme, wie z.B. Pyruvatkinase M2 (PK-M2), Enolase, Glycerinaldehyd-3-Phosphat-Dehydrogenase (GAPDH) und Lactatdehydrogenase, aber auch ein Teil der zellulären Nukleosiddiphosphatkinase (NDPK) A, Adenylatkinase, einige Proteinkinasen und AU-reiche mRNA-Spezies (Mazurek et al., *Biochem J* 356: 247-256 (2001); Mazurek et al., *J. Cell Physiol*. 167: 238-250 (1996); Mazurek et al., *Anticancer Res.* 18: 3275-3282 (1998)).

NDPK A ist das Genprodukt des nm23-H1-Gens, welches als putatives Metastasierungssuppressorgen isoliert wurde. Beispielsweise aus der Literaturstelle A. Leone et al., Cell 65:25-35 (1991) ist bekannt, dass das humane nm23-Gen nm23-H1 bei Überexpression in Tumorzellen deren Metastasierungspotential beachtlich reduziert. Nm23-H1 scheint daher ein Metastasierungssuppressorgen zu sein. Zur Familie gehört neben weiteren Genen auch nm23-H2. Die Expressionsprodukte dieser Gene sind bekannt als NDPK A bzw. NDPK B (Nukleosiddiphosphatkinase A bzw. B). Im Glykolyseenzymkomplex wurde nur die saure Isoform NDPK A gefunden, nicht aber die NDPK B, welche einen basischen Isoelektrischen Punkt hat.

In Übereinstimmung ist aus der Literaturstelle M. Engel et al., J. Biol. Chem. 273(32): 20058-20065 (1998) bekannt, dass NDPK A - nicht aber NDPK B - teilweise mit dem Glykolyseenzym GAPDH assoziiert vorliegt, welches in der Glykolyse Glycerinaldhehyd-3-Phosphat zu 1,3-Bisphosphoglycerat umsetzt. In vitro wird NDPK A (Nm23-H1-Protein)im Komplex mit GAPDH zur Phosphorylierung anderer Proteine aktiviert.

In anderen Zusammenhängen sind die Peptide Lys-Tyr-Pro-Glu und Lys-Tyr-Ala-Glu aus den Literaturstellen WO 01/04316 und WO 00/05250 bekannt.

Darüber hinaus enthält die Proteinsequenz der Alpha-ribazol-5'-phosphat-Phosphatase aus E.coli die Aminosäurefolge "Trp-Leu-Ile-Arg-His-Gly-Glu" (Swissprot Accession No. P52086).

### Technisches Problem der Erfindung.

Der Erfindung liegt das technische Problem zu Grunde, Wirkstoffe für eine pharmazeutische Zusammensetzung oder Screeningverfahren für die Isolierung solcher Wirkstoffe anzugeben, welche den Energiestoffwechsel von Tumorzellen, Leukämiezellen oder anderen unkontrolliert proliferierenden Zellen spezifisch stört und so die unphysiologische Proliferation der kranken Zellen effektiv unterbindet, ohne dass gesunde Zellen geschädigt werden.

Grundzüge der Erfindung und bevorzugte Ausführungsformen.

Als eine Lösung dieses technischen Problems lehrt die Erfindung ein Protein oder Peptid mit einer Aminosäurensequenz gemäß Seq.-ID 1 oder eine hierzu homologe Aminosäurensequenz, nicht jedoch PGM B. Zu erfindungsgemäßen Proteinen gehören auch nicht aktivierbare oder nicht aktive Mutanten von PGM.
Die Erfindung beruht auf der in den Ausführungsbeispielen näher erläuterten Erkenntnis, dass eine Aktivierung von PGM in Tumorzellen dadurch erfolgt, dass PGM durch einen Komplex NDPK A/GAPDH oder durch einen anderen Mechanismus, an dem NDPK A beteiligt ist, im Bereich der angegebenen Sequenz phosphoryliert und so aktiviert wird. Diese Erkenntnis ist zu der technischen Lehre umgesetzt, die Aktivierung von PGM dadurch zu reduzieren oder gar ganz zu inhibieren, indem der Proteinkinase-aktiven Form von NDPK A/Nm23-H1 im Komplex als kompetitives Substrat ein erfindungsgemäßes Protein oder Peptid angeboten wird, mit der Folge, dass für die Aktivierung von PGM eine zumindest reduzierte Menge des aktiven Nm23-H1 Proteins zur Verfügung steht.

Weiterhin ist die oben genannte Erkenntnis zu der technischen Lehre umgesetzt, ein PGMenthaltendes Präparat aus Tumorzellen oder aus rekombinanter Herstellung so in einem Screeningassay einzusetzen, dass Hemmstoffe aus einer getesteten Serie von Kandidatenverbindungen isoliert werden, die die PGM-Aktivität spezifisch nur dann effizient hemmen, wenn die zellulären Gehalte des Aktivators 2,3-BPG niedrig sind, wie z.B. in proliferierenden Tumorzellen. So identifizierte organische Verbindungen würden das erfindungsgemäße Peptid in seiner Wirkung nachahmen, bei gleichzeitigen Vorteilen in den pharmakologischen Eigenschaften, wie z.B. orale Verfügbarkeit, geringere Immunogenität, größere Stabilität, z.B. gegen Proteolyse.

Für eine Wirkung des erfindungsgemäßen Peptids oder Proteins muss dieses in das Zytoplasma einer Zelle transportiert werden. Je nach Größe des erfindungsgemäßen Moleküls wird es sich daher empfehlen, wenn das Protein oder Peptid über seine freie Aminogruppe mit einem Transportpeptid oder Transportmolekül verbunden wird. Durch dieses Transportpeptid oder Transportmolekül erfolgt ein effektiver Transport durch die Zellmembran in das Zytoplasma. Grundsätzlich sind alle für humane Zellen geeignete Transportsysteme im Rahmen der Erfindung einsetzbar. Beispielsweise ist ein Reagenz namens Chariot® kommerziell erhältlich (Firma ActiveMotif), welches mit Proteinen oder Peptiden nichtkovalente Komplexe bildet, die membrangängig sind. Zu solchen Transportsystemen gehören auch transmembrane Proteine oder Peptide, wobei dann die erfindungsgemäße Sequenz an einen membranaußenständigen Teil des transmembranen Proteins oder Peptids fusioniert ist. Lediglich beispielhaft wird für Transportsubstanzen die Gruppe bestehend aus "Lectine, synthetische Polymere, Zucker, Riboflavin, Antennapedia-Homöodomäne-Peptid von Drosophila, HIV-tat-Peptid, EnterotoxinB-Protein von E.coli, Importpeptid-Carrier, Arginin-Oligomere, VP22" genannt. Hierzu wird ergänzend auf die Literaturstellen C.-M. Lehr et al., J. Controlled Release 65:19-29 (2000), G. Cantuaria et al., Cancer 88:381-388 (2000), S.R. Holliday et al., Biochim Biophys Acta 1426:195-204 (1999), D. Derossi et al., J. Biol. Chem. 269:10444-10450 (1997), A. Marcello et al., Biochem Soc Trans 20:311S (1992), S. Dokka et al., Pharm. Res. 14:1759-1766 (1997), und J. Rothbart et al., Nature Medicine 6:1253-1257 (2000) verwiesen. Als gut geeignet hat sich als Transportpeptid eine Aminosäurensequenz gemäß Seq.-ID 2 oder eine Teilsequenz mit zumindest 4 aufeinander folgenden Aminosäuren hieraus erwiesen (siehe auch D. Derossi et al., J. Biol. Chem. 271:18188-18193 (1996), wobei dieses die besagte Sequenz aufweist oder daraus besteht.

Zum Zwecke der einfachen Gewinnung hochreiner erfindungsgemäßer Wirkstoffe kann es sich empfehlen, wenn das Protein oder Peptid mit einem geeigneten und physiologisch verträglichen Affinitätstag versehen wird. Beispielsweise kann die Aminogruppe oder die Carboxylgruppe der Seq.-ID 1 mit einem Reinigungsaffinitätspeptid verbunden sein.

Beispielsweise kann das Reinigungspeptid eine Aminosäurensequenz gemäß Seq.-ID 3 oder eine Teilsequenz mit zumindest 4 aufeinanderfolgenden Aminosäuren hieraus aufweisen oder daraus bestehen. Die Histidingruppen sind in der Lage Nickelion-Chelate zu bilden, so dass eine Reinigungsäule mit immobilisiertem Nickelionen (Ni2+) eingesetzt werden kann.

Zur Erhöhung der Aktivität und folglich zur Verbesserung der kompetitiven Bindung des nm23-H1/GAPDH Komplexes kann es sich empfehlen, wenn zwischen der Aminosäurensequenz gemäß Seq.-ID 1 und dem Transportpeptid ein Spacermolekül eingebaut ist. Als Spacermoleküle kommen grundsätzlich alle physiologisch verträglichen Gruppen in Frage. Bei geeigneter Applikation, beispielsweise intraläsional, braucht die Gruppe jedoch nicht notwendigerweise physiologisch verträglich zu sein; entsprechendes gilt für ein Affinitätstag. Ein synergistischer Effekt wird erreicht, wenn das Spacermolekül eine Aminosäurensequenz gemäß Seq.-ID 3 oder eine Teilsequenz mit zumindest 4 aufeinanderfolgenden Aminosäuren hieraus aufweist oder hieraus besteht, da das Spacermolekül dann zugleich als Affinitätstag eingesetzt werden kann.

Eine besonders effektive kompetitive Bindung wird erreicht, wenn ein Peptid oder Protein eingesetzt wird, welches eine Aminosäurensequenz gemäß Seq.-ID 4 enthält oder daraus besteht.

Ein erfindungsgemäßes Protein oder Peptid kann die erfindungsgemäßen Sequenzen gemäß der angegebenen Sequenzprotokolle jeweils einfach oder mehrfach enthalten. Auch ist beispielsweise eine Dimerisierung möglich, wobei mehrere Sequenzen nach Seq.-ID 1 in einem Molekül oder in verschiedenen Molekülen die Dimere bilden können. Auch ist eine Zyklisierung eines erfindungsgemäßen Proteins oder Peptids möglich, was die Transmembrangängigkeit per se verbessert. Im Falle großer Peptide bzw. von Proteinen kann sich eine Denaturierung, beispielsweise mittels chaotroper Lösungsmittel, empfehlen, da ungefaltete Strukturen per se eine bessere Transmembrangängigkeit aufweisen und eine eigene enzymatische Aktivität des eingesetzten Proteins oder Peptids nicht erforderlich ist für die kompetitive Bindung.

Die Erfindung lehrt weiterhin eine Nukleinsäure kodierend für ein erfindungsgemäßes Protein oder Peptid einen Vektor enthaltend eine solche Nukleinsäure sowie eine Zelle, welche mit einem solchen Vektor transformiert ist. Mit solchen Zellen lassen sich erfindungsgemäße Proteine und Peptide auf einfache Weise rekombinant herstellen. Alternativ kann eine Herstellung durch Isolierung von PGM und Proteolyse erfolgen. Schließlich ist auch die Herstellung in einem zellfreien Proteinbiosynthesesystem möglich, welches dann eine für ein erfindungsgemäßes Protein oder Peptid codierende RNA enthält. Selbstverständlich ist insbesondere im Falle kurzer Peptide die chemische Synthese möglich.

Mit einem erfindungsgemäßen Protein oder Peptid läßt sich eine pharmazeutische Zusammensetzung zur Behandlung von Krebs, insbesondere von bösartigen Neoplasien, herstellen. Daher lehrt die Erfindung weiterhin eine Pharmazeutische Zusammensetzung, insbesondere zur Behandlung von Krebs, enthaltend ein erfindungsgemäßes Protein oder Peptid in physiologisch wirksamer Dosis, vorzugsweise in galenischer Herrichtung zur i.v., i.p., subkutanen oder intraläsionalen Injektion. Schließlich lehrt die Erfindung ein Verfahren zur Behandlung von Krebs, wobei einem Patienten eine pharmazeutische Zusammensetzung nach Anspruch 31 in einem definierten Behandlungsplan dargereicht wird.

Da sich ein das wirkungslose Kontrollpeptid (MRQIKIWFPNRRMKWKKHHHHHHPWRIEGHL) von einem erfindungsgemäßen Peptid (Seq.-ID 4) nur in einer Sequenz von 5 Aminosäuren unterscheidet, ist es nahe liegend, dass sich auch peptidomimetische Substanzen oder andere kleine organische Verbindungen ausgehend von der spezifischen Sequenz entwickeln lassen, welche die Wirkungsweise eines erfindungsgemäßen Peptides nachahmen und darüber hinaus einige Vorteile besitzen, wie z.B. per orale Administrierbarkeit, niedriges immunogenes Potential und höhere Affinität zur Targetstruktur.

Zur Entwicklung einer selektiven, kleinen organischen Verbindung als Hemmstoff der PGM, in bevorzugter Ausführung als Hemmstoff des Aktivierungsmechanismus der PGM in Tumorzellen, wird durch die Erfindung ein Verfahren zur Identifizierung und Testung der prospektiven Wirkstoffe beschrieben. Eine mögliche Ausführung betrifft die Verwendung von Nm23-H1 und/oder GAPDH oder einer Teilsequenz hieraus (RNA oder Protein bzw. Peptid oder nm23-H1/GAPDH-Komplex) zum Screenen nach kompetitiv mit PGM daran bindenden Substanzen oder nach Substanzen, welche die Bildung von nm23-H1 und/oder GAPDH und/oder des nm23-H1/GAPDH-Komplexes inhibierenden oder inaktivierenden, insbesondere prospektiven Wirkstoffen zur Inhibierung von besagter RNA oder besagtem Protein oder Peptid oder Komplex, wobei eine prospektive Substanz oder eine Mischung solcher prospektiver Substanzen mit besagter RNA oder besagtem Protein oder Peptid oder Komplex kontaktiert wird, wobei mit einem Bindungsassay Bindungsereignisse festgestellt werden (beispielsweise durch Bestimmung der Abnahme der Aktivität von endogenem oder zugegebenem PGM oder durch Bestimmung der Abnahme der Mengen an nm23-H1 und/oder GAPDH und/oder nm23-H1/GAPDH-Komplex in einem in vitro- oder in vivo-System), und wobei eine bindende prospektive Substanz, ggf. nach Dekonvolutierung, selektiert wird. Ein solches Screening kann in der gleichen Ausführungsform auch zur Identifizierung von prospektiven Detektorsubstanzen dienen, die sich als Reportersubstanzen in quantitativen Assays einsetzen lassen.

Eine andere Ausführung beschreibt die Verwendung von PGM oder eines PGM-enthaltenden zytosolischen Zellisolates oder des isolierten Glykolyseenzymkomplexes zum Screenen nach Verbindungen, welche die mittels einer enzymatischen Messkette gemessene PGM-Aktivität oder die Aktivierbarkeit der PGM hemmen, oder welche die Phosphoglycerat-Phosphatase-Aktivität der PGM erhöhen, die unter normalen zellulären und in-vitro-Bedingungen niedrig ist, aber beispielsweise in Anwesenheit von Orthovanadat erheblich gesteigert wird (Stankiewicz et al., Biochemistry 26: 1264-1269 (1987)). Eine verstärkte Phosphatase-Aktivität führt in Abwesenheit eines kontinuierlichen Rephosphorylierungsmechanismus für das aktive Zentrum zu einer Deaktivierung der PGM, die ebenfalls als Abnahme der PGM-Enzymaktivität gemessen werden kann. In der Ausführung als Screening auf Aktivatoren der Phosphoglycerat-Phosphatase-Aktivität der PGM wird in der bevorzugten Ausführung kein Orthovanadat zugegeben und wahlweise auch kein dCTP; weiterhin kann der Assay auch mit gereinigter und/oder rekombinant hergestellter PGM durchgeführt werden.
Der Aufbau eines gekoppelten enzymatischen Assays erfolgt bevorzugt unter Verwendung der 3-Phosphoglyceratdehydrogenase (3-PGDH), welche das von der PGM aus 2-Phosphoglycerat gebildete 3-Phosphoglycerat weiter umsetzt zu 3-Phosphohydroxypyruvat, wobei aus NAD+ NADH gebildet wird, was in einer messbaren Zunahme der UV-Absorption bei 340 nm resultiert. Die für den Assay erforderliche 3-PGDH kann von beliebigen Spezies stammen solange Kompatibilität mit den Assaybedingungen gewährleistet ist. Um eine effiziente Expression in guter Ausbeute in E.coli als Wirtszelle zu ermöglichen, kann die kodierende 3-PGDH-Sequenz aus E.coli DNA in einen Expressionsvektor kloniert, in E.coli überexprimiert und z.B. mittels eines His-Tag gereinigt werden. Darüber hinaus ist 3-Phosphoglyceratdehydrogenase kommerziell erhältlich, z.B. von Sigma-Aldrich Chemicals (Sigma Kat.-Nr. P4579).
Alternativ kann auch eine Enzymkette bestehend aus Enolase, Pyruvatkinase und Lactatdehydrogenase zur Messung der PGM-Aktivität über den NADH-Verbrauch der Lactatdehydrogenase verwendet werden. Alle benötigten Enzyme sind von verschiedenen Spezies und in geeigneter Reinheit z.B. von den Firmen Sigma, Fluka oder Roche Diagnostics erhältlich.
Als bevorzugte Quelle für die PGM-Aktivität beschreibt die Erfindung die lösliche, cytoplasmatische Fraktion von Tumorzellen, z.B. aus der MCF-7-Zelllinie, die durch Dialyse gegen eine Suspension von Aktivkohle und Polyethylenimin von endogenen Phosphometaboliten befreit wird, oder, noch bevorzugter, die durch Isoelektrische Fokussierung von cytoplasmatischem Tumorzellextrakt erhaltenen Fraktionen des Glykolyseenzym-Komplexes, die dadurch gekennzeichnet ist, dass in diesen Fraktionen die Enzymaktivitäten von PGM, NDPK A und GAPDH kofokussieren. Als Aktivatorsubstanzen, die die "primende" Phosphorylierung des aktiven PGM-Zentrums vermitteln, können bei Verwendung des Prä-Glykolyseenzymkomplexes aus Tumoren oder Tumorzellinien auch Nukleotide fungieren, deren terminale Phosphatgruppen unter Beteiligung der NDPK A - entweder direkt oder unter Einbeziehung eines oder mehrerer weiterer Proteine - auf die PGM übertragen werden. In der bevorzugten Ausführung wird als Nukleotid dCTP verwendet, welches eine weitgehende Spezifität für die NDPK A bereitstellt. dCTP wirkt nur in der Komplexumgebung als Aktivator der PGM, während dCTP - im Gegensatz zu 2,3-BPG - von der gereinigten PGM nicht als Phosphorylierungsdonor verwendet werden kann. Durch die Zugabe von dCTP werden die Assaybedingungen so eingestellt, dass Hemmstoffe für den Nukleosidtriphosphatabhängigen Aktivierungsweg der PGM identifiziert werden können. Auf diese Weise selektierte Verbindungen imitieren die Wirkungsweise eines erfindungsgemäßen Peptids (Seq.-ID 4). Aus der Literaturstelle Repiso A, et al., Haematologica 88: ECR07 (2003) ist bekannt, dass eine genetisch bedingte 50%ige Erniedrigung der PGM B-Aktivität in allen Zellen des menschlichen Körpers abgesehen von Symptomen wie Müdigkeit oder Anämie vom Organismus toleriert wird, so dass auch ein nicht völlig tumorspezifischer Hemmstoff für die PGM B von Patienten auch bei einer längerfristigen Behandlung noch gut verträglich sein dürfte.

Erfindungsgemäß führt daher eine Verwendung der PGM enthaltenden Fraktionen in den beschriebenen Ausführungen zur Identifikation von Verbindungen, die die Aktivierbarkeit durch Nukleosidtriphosphate hemmen, oder die intrinsische Phosphataseaktivität der PGM erhöhen, oder ein anderes Enzym innerhalb der Messkette hemmen, oder durch Bindung im aktiven Zentrum der PGM kompetitiv die Subtratumsetzung oder -bindung hemmen. Um letztere, unerwünschte Gruppe von Inhibitorsubstanzen auszusondern, werden alle identifizierten Verbindungen mit der zuvor verwendeten Methode erneut gescreent, diesmal jedoch in Anwesenheit von 2,3-BPG und/oder 1,3-BPG, bevorzugt in mehreren Ansätzen mit verschiedenen Konzentrationen. Als prospektiv tumorspezifische Wirkstoffe gemäß der Erfindung sind solche Verbindungen zu werten, deren im ersten Screening festgestellte PGM-Hemmwirkung durch die Zugabe von 2,3-BPG aufgehoben wird, was bei den Verbindungen, die an das aktive Zentrum binden, nicht der Fall ist. Die beschriebene Kombination von Screening mit und ohne 2,3-BPG kann auch parallel in einer Screeningrunde durchgeführt werden und selektiert Verbindungen, deren inhibitorisches Profil zu dem des erfindungsgemäßen Peptids analog ist, da die Hemmwirkung des letzteren durch 2,3-BPG kompensiert (= aufgehoben) wird. Verbindungen, die ein anderes Enzym als PGM in der gewählten Messkette hemmen, können einfach dadurch ausgesondert werden, dass man das jeweilige Screening wiederholt, unter Ersatz der PGM durch das Produkt der PGM-Reaktion.

Während die oben beschriebenen Ausführungen der Erfindung ein therapeutisch wirksames inhibitorisches Protein oder Peptid, oder ein Verfahren zur Identifikation von analog wirksamen Verbindungen angeben, deren Toxizität gegenüber normalen Zellen aufgrund der dort vorhandenen höheren Gehalte an kompensatorisch wirksamem 2,3-BPG gering ist, nutzt ein weiterer Aspekt der Erfindung die Einbringung oder Erhöhung von 2,3-BPG in den als 2,3-BPG-defizient bekannten stark proliferierenden Zellen als therapeutischer Ansatz.

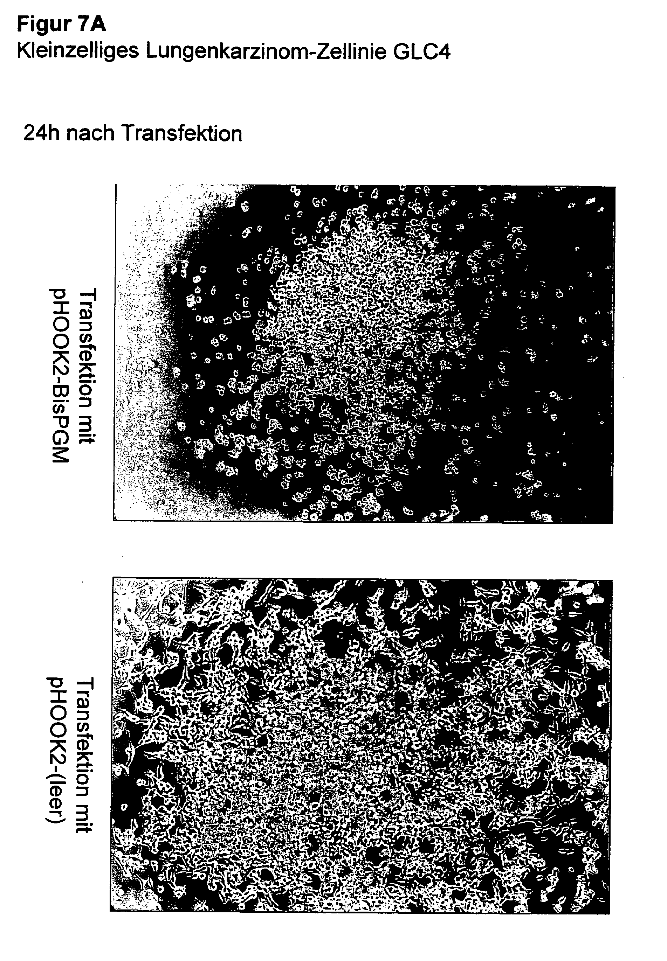

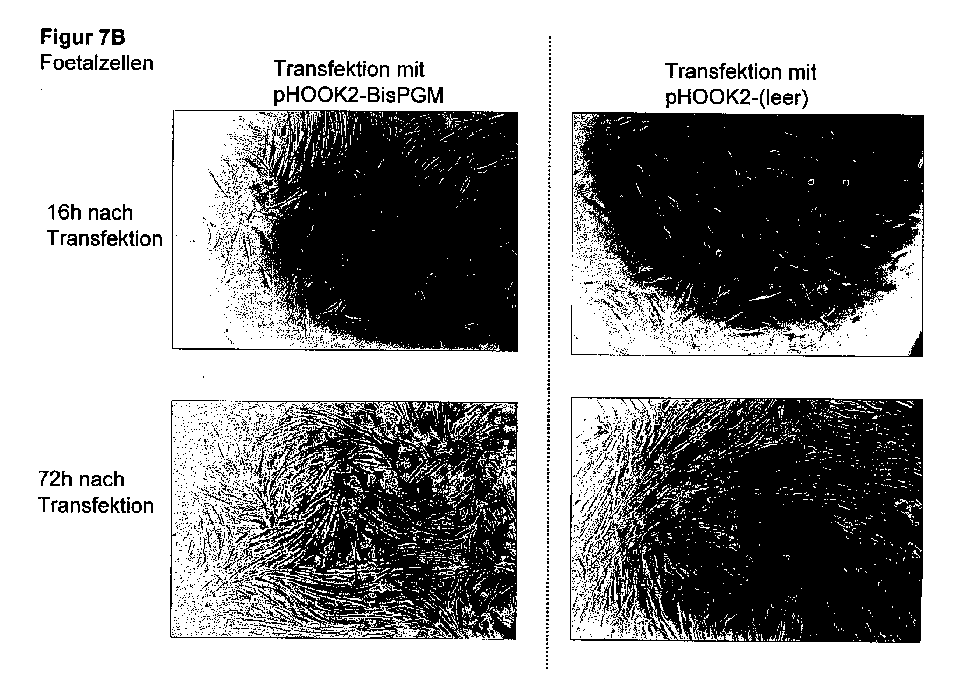

Wie in einem Ausführungsbeispiel (Figur 7) dargestellt, führt das Einbringen eines Expressionsvektors mit der kodierenden Sequenz der BisPGM in eine Tumorzelllinie zum Zelltod, während normale Embryonalzellen, die mit dem BisPGM-Expressionsvektor transfiziert werden, lediglich mit einer Reduktion der Zellteilungsrate reagieren.
Weitere Ausführungen der Erfindung betreffen daher Methoden zur Erhöhung der 2,3-BPG-Spiegel in unkontrolliert proliferierenden Zellen und Zellverbänden, die im folgenden aufgezählt werden:
a) Einschleusung von expressionsfähigen Nukleinsäurekonstrukten, beispielsweise in Form von Plasmid-DNA oder viralen DNA-Vektoren, enthaltend die komplette kodierende Sequenz der BisPGM (Swissprot accession No. P07738) oder ein Teilstück daraus, in die Zielzellen, so dass die BisPGM-Sequenzen dort exprimiert werden. Zielzellen können behandlungsbedürftige Zellen oder Zellgewebe in einen Organismus sein oder auch Zellen, welche mit einem erfindungsgemäßen Vektor transformiert sind zum Zwecke der rekombinanten Protein- oder Peptidherstellung.
b) Behandlung der Zielzellen oder der Zellverbände durch die Verabreichung einer Verbindung, die die endogen vorhandende BisPGM durch Bindung an die BisPGM oder durch die Aufhebung von spezifischen Hemmmechanismen aktiviert oder die Expression der BisPGM durch spezifische Aktivierung von Transkriptions- und/oder Translationsvorgängen steigert.
c) Verabreichung des rekombinanten oder aus Zellmaterial aufgereinigten BisPGM-Proteins oder eines oder mehrerer Peptide, die Teilsequenzen der BisPGM darstellen und hergestellt werden können durch chemische Synthesen, rekombinante Expressionstechniken, Verwendung eines zellfreien Proteinbiosynthesesystems welches dann die für ein erfindungsgemäßes Peptid oder Protein kodierende RNA oder DNA enthält, oder proteolytische Behandlung der BisPGM. Bevorzugterweise sind die genannten Proteine oder Peptide kovalent oder nicht-kovalent gebunden an ein Transportmolekül oder -system, welches die Zellgängigkeit und/oder orale Bioverfügbarkeit der BisPGM-Proteine oder -peptide vermitteln, wie oben bereits beschrieben für Peptide enthaltend eine Teilsequenz aus Seq.-ID 1. Im Falle großer Peptide bzw. des kompletten Proteins kann sich eine Denaturierung, beispielsweise mittels chaotroper Lösungsmittel, empfehlen, da ungefaltete Strukturen per se eine bessere Transmembrangängigkeit aufweisen.
d) Therapeutische Verabreichung von 2,3-BPG selbst sowie von chemischen Analoga dieser Verbindung. Als Analogverbindungen des 2,3-BPG können die 2,3-Bis(sulfoamino)propionsäure, die 2,3-Bisphosphonopropionsäure oder die 2-Carboxybutan-1,4-bisphosphonsäure synthetisiert und verwendet werden; die Synthese der letzten beiden Verbindungen wurde beschrieben in Pfeiffer et al., J. Med. Chem. 17 (1974) 112-115. Die erste Verbindung ist leicht aus der Reaktion von 2,3-Diaminopropionsäure und Chlorsulfonsäure zugänglich. Alle vorgenannten Verbindungen sind Strukturanaloge des 2,3-BPG, sind aber stabiler gegen enzymatische Hydrolyse. Durch den in der Medizinalchemie allgemein bekannten Ersatz der Carboxylatgruppe durch die bioisostere Tetrazol-Gruppe lassen sich zudem Verbindungen synthetisieren, die mit höherer Wahrscheinlichkeit Zellmembranen passieren können. Im vorliegenden Fall würde bevorzugt auf der Basis der 2,3-Bis(sulfoamino)propionsäure die Verbindung 1,2-Bis(sulfoamino)-1-(1*H*-tetrazol-5-yl)ethan synthetisiert werden.

Mit einem erfindungsgemäßen Protein, Peptid, einer dafür kodierenden Nukleinsäure oder 2,3-BPG läßt sich eine pharmazeutische Zusammensetzung zur Behandlung von Krebs, insbesondere von bösartigen Neoplasien, herstellen. Daher lehrt die Erfindung weiterhin eine Pharmazeutische Zusammensetzung, insbesondere zur Behandlung von Krebs, enthaltend ein erfindungsgemäßes Protein oder Peptid in physiologisch wirksamer Dosis, vorzugsweise in galenischer Herrichtung zur i.v., i.p., subkutanen oder intraläsionalen Injektion. Schließlich lehrt die Erfindung ein Verfahren zur Behandlung von Krebs, wobei einem Patienten eine pharmazeutische Zusammensetzung nach Anspruch 31 in einem definierten Behandlungsplan dargereicht wird.

Die Erfindung stellt auch weitere Verfahren zur Verfügung, welche zur Identifikation von therapeutisch nutzbaren Verbindungen geeignet sind. Ein Verfahren besteht darin, dass in einem Assay ausgiebig dialysierter cytoplasmatischer Extrakt aus Zellen oder Gewebe, Subfraktionen eines solchen Extraktes oder aus Zellen oder Gewebe geeinigte BisPGM oder rekombinant hergestellte BisPGM mit Testverbindungen in Kontakt gebracht werden und durch Messung der BisPGM-Aktivität die BisPGM-aktivierenden Verbindungen identifiziert werden. Eine Messung der BisPGM-Aktivität in einem homogenen Assay kann beispielsweise erfolgen durch eine Kopplung mit der GAPDH-Reaktion, wie beschrieben von Ravel et al., C.R. Acad. Sci. Paris, Sciences de la vie 320: 27-33, (1997).
Ein weiteres erfindungsgemäßes Verfahren lehrt die Identifizierung von Substanzen welche die Transkriptionsrate und/oder Translationsrate des BisPGM-Gens erhöhen. Zu diesem Zweck wird ein nach dem Stand der Technik gebräuchlicher Reportergen-Assay eingesetzt, wobei die Promotorsequenz (genomische 5'-flankierende Region, Genbank Accession No. M23066 (776 bp), längere 5'-Sequenz publiziert in gi|11120834|gb|AC083874.2|AC083874 (Homo sapiens chromosome 7 clone RP11-792L21)) und wahlweise auch die 3-untranslatierte Region des BisPGM-Gens (793 bp, z.B. publiziert in Accession No. BC017050) in einem Plasmid- oder viralem Nukleinsäurevektor vor ein Reportergen kloniert wird, beispielsweise kodierend für ein fluoreszierendes Protein oder für Luciferase. Anschließend werden mit dem Vektorkonstrukt transfizierte eukaryontische, bevorzugt menschliche Zellen für ein Screening auf Verbindungen verwendet, welche die Aktivität des Reportergens erhöhen, im genannten Beispiel also einen Anstieg der gemessenen Fluoreszenz bzw. Lumineszenz verursachen. Ein solches Screening lässt sich auch im Hochdurchsatzverfahren durchführen (Goetz et al., J. Biomol. Screen. 5: 377-384 (2000)). Üblicherweise werden aus den zunächst identifizierten Verbindungen solche herausgefiltert, welche die Genaktivität noch vieler anderer Gene erhöhen. Bevorzugt im Rahmen dieser Erfindung ist zunächst ein Aussortieren solcher Verbindungen, welche in einem Reportergen-Assay auch den PGM-Promotor einschalten (Accession No. AL355490 (BAK clone RP11-452K12)).

Darüber hinaus ist als weitere Ausführung auch die Entwicklung künstlicher Transkriptionsfaktoren möglich, welche spezifische Elemente im BisPGM-Promotor erkennen und nach Bindung die Transkriptionsrate erhöhen. Aus einer Publikation ist beispielsweise bekannt, dass sich Zinkfinger-Motive bestimmter Transkriptionsfaktoren durch rationales Design und/oder kombinatorische Methoden für einen beliebigen Genpromotor maßschneidern lassen (Bae et al., Nature Biotechnology 21: 242-243 (2003), US2003/0165997A1).

Sämtliche aufgezählten Methoden schließen alle mögliche Verabreichungsformen für den zu behandelnden Organismus ein, beispielsweise die orale Verabreichung der Verbindungen oder die systemische oder intraläsionale Injektion.

Folgend werden weitere Aspekte der Erfindung, welche von selbstständiger Bedeutung sind, näher erläutert.

Weitere Aspekte der Erfindung betreffen die spezielle, in vitro nachgewiesene Proteinkinaseaktive Form des Nm23-H1/NDPK A-Proteins, welche wie aus der Literatur bekannt, durch die Assoziierung mit GAPDH erzeugt wird (M. Engel et al., J. Biol. Chem. 273(32):20058-20065 (1998)).
Es wurde gefunden, dass der nm23-H1/GAPDH Komplex in Tumorgewebe, insbesondere in Lungentumor-, Brusttumor- und Colontumorgewebe, differenziell hochreguliert ist. Diese Erkenntnis führt zu folgenden Lehren:
i) Verwendung einer nm23-H1 und/oder GAPDH (RNA oder Protein bzw. Peptid oder nm23-H1/GAPDH-Komplex) bindenden Substanz zur Detektion von Krebs, und/oder von Metastasen aus Primärtumoren, oder zur Detektion eines Risikos der Erkrankung an Krebs, wobei eine Gewebeprobe in vitro oder in vivo auf Transkription oder Übertranskription von nm23-H1 und/oder GAPDH RNA oder auf Expression oder Überexpression des nm23-H1 und/oder GAPDH Proteins (ggf. im Komplex) untersucht wird, ggf. standardisiert gegen eine Normalgewebeprobe des gleichen oder eines verschiedenen Patienten, wobei die Detektorsubstanz vorzugsweise eine Reportergruppe enthält, wobei Bindung von nm23-H1 und/oder GAPDH an die Detektorsubstanz halbquantitativ oder quantitativ detektiert wird;
ii) Verfahren nach(i), dadurch gekennzeichnet dass eine Detektorsubstanz in einer Ausführungsform mit einer Reportergruppe in zu untersuchendes Gewebe (in vivo oder in vitro) appliziert wird, wobei das zu untersuchende Gewebe dann einer Detektionsverfahrenstufe unterworfen wird, welche sensitiv für die Reportergruppe ist, und wobei im Fall der Detektion eines definierten Mindestwertes der Reportergruppe im Gewebe das Gewebe als Tumorzellen enthaltend qualifiziert wird (alternativ kann ein Peptid oder Protein gemäß einem der Ansprüche 1 bis 9, vorzugsweise versehen mit einem Affinitätstag, beispielsweise (His)6, Strep-Tag, Biotin, Gluthathion-S-Transferase, Maltose-bindendes Protein, Calmodulin, mit einem Testlysat aus tumorverdächtigen Zellen kontaktiert werden, wobei das Peptid oder Protein als Substrat der endogenen nm23-Proteinkinaseaktivität beispielsweise entweder durch ein zugegebenes γ32P-Nukleosidtriphosphat radioaktiv phosphoryliert wird und der Einbau ggf. nach Binden des Peptids an eine feste Matrix und Waschen quantifiziert wird, oder die Menge an phosphoryliertem Peptid oder Protein nach Einbau von nicht-radioaktivem Phosphat durch Binden des Peptids an beispielsweise ein Eisenchelat bestimmt wird, wobei das Peptid oder Protein mit einer Reportergruppe versehen bzw. mit einer Reportersubstanz gekoppelt ist);
iii) Verwendung an nm23-H1 und/oder GAPDH (RNA oder Protein bzw. Peptid oder nm23-H1/GAPDH-Komplex) bindenden Substanz zur Herstellung einer pharmazeutischen Zusammensetzung zur Behandlung von Krebs und/oder Metastasen aus Primärtumoren (die Substanz kann ein vorstehend beschriebenes Protein oder Peptid, ein Antikörper, welcher durch Immunisierung eines nicht-menschlichen Säugetiers mit einem nm23-H1 und/oder GADPH Peptid oder Protein, oder einer hierfür codierenden cDNA, erhältlich ist, oder ein Phage-Display Antikörper sein; es kann sich auch um eine Mimikriverbindung eines solchen Antikörpers handeln, beispielsweise eine Teilsequenz aus der variablen Region; es kann sich um ein Aptamer, eine antisense RNA, oder ein Ribozym gegen nm23-H1 und/oder GADPH handeln; die Substanz kann zusätzlich eine zytotoxische und/oder immunstimulierende Komponente tragen).

### Definitionen.

Im Rahmen dieser Beschreibungen werden die Bezeichnungen PGM (Phosphoglyceratmutase) und BisPGM (Bisphosphoglyceratmutase) für alle nicht-humanen und humanen Isoformen benutzt (beispielsweise PGM B) oder neu, auf Nukleinsäuren- oder Aminosäurenbasis, verwendet. Mit diesen Begriffen mit umfaßt sind auch die beispielsweise im Rahmen dieser Beschreibung offenbarten kurzen Sequenzen, welche aus einer Isoform stammen. Weiterhin mit umfaßt sind auch Homologe, wobei die Homologie zumindest 80%, vorzugsweise mehr als 90%, höchstvorzugsweise mehr als 95%, beträgt. Im Falle der Nukleinsäuresequenzen sind auch komplementäre oder allelische Varianten mit umfaßt.

Homologie liegt insbesondere vor, wenn in der Sequenz gemäß Seq.-ID 1 folgende Substitutionen vorliegen: i) L-Arginin ersetzt durch D-Arginin, L-Lysin, D- Lysin, homo-Arginin, D-homo-Arginin, Methionin, D-Methionin, Ornithin oder D-Ornithin, ii) L-Histidin ersetzt durch D-Histidin, 1-Serin, D-Serin, homo-Serin, Threonin, D-Threonin, allo-Threonin, Cystein, D-Cystein, Tyrosin, oder D-Tyrosin, iii) Glycin ersetzt durch L-Alanin, D-Alanin, L-Prolin, D-Prolin, beta-Alanin, iv) L-Glutaminsäure ersetzt durch D-Glutaminsäure, L-Asparaginsäure, D-Asparaginsäure, L-Asparagin, D-Asparagin, L-Glutamin oder D-Glutamin. Homologie liegt jedoch nicht vor im Falle der Sequenzen Lys-Tyr-Pro-Glu oder Lys-Tyr-Ala-Glu.

Weiterhin sind Sequenzen der PGM oder BisPGM einbegriffen, welche lediglich Teilsequenzen der explizit offenbarten Sequenzen, beispielsweise ein Exon oder mehrere Exons, oder komplementärer Sequenzen hierzu darstellen, mit der Maßgabe, dass diese Teilsequenzen im Falle der Nukleinsäuren eine für eine Hybridisierung mit einer erfindungsgemäßen Nukleinsäure hinreichende Länge, zumindest 50 Basen, aufweisen und im Falle der Proteine bzw. Peptide mit zumindest gleicher Affinität an ein protein- oder peptidspezifisches Zielmolekül binden. Weiterhin sind alle mit erfindungsgemäßen Nukleinsäuren hybridisierende Nukleinsäuren umfaßt, nämlich solche, die unter stringenten Bedingungen (5°C bis 25°C unterhalb der Aufschmelztemperatur; siehe ergänzend J.M. Sambrook et al., A laboratory manual, Cold Spring Harbor Laboratory Press (1989) und E.M. Southern, J Mol Biol, 98:503ff (1975)) hybridisieren. Es versteht sich, dass die Erfindung auch Expressionskassetten umfaßt, i.e. eine oder mehrere der erfindungsgemäßen Nukleinsäuresequenzen mit mindestens einer Kontroll- oder regulatorischen Sequenz. Eine solche Expressionskassette kann auch eine Sequenz für ein bekanntes Protein umfassen, wobei im Zuge der Translation ein Fusionsprotein aus einem bekannten Protein und einem erfindungsgemäßen Protein oder Peptid entsteht. Schließlich sind RNA sowie damit korrelierende DNA und umgekehrt umfaßt, ebenso wie genomische DNA als auch korrelierte cDNA und umgekehrt.

Im Zusammenhang mit erfindungsgemäßen Verwendungen umfassen die Begriffe der PGM oder BisPGM Nukleinsäuren oder Proteine bzw. Peptide neben den Volllängen der offenbarten Sequenzen (siehe auch vorstehender Absatz) auch Teilsequenzen hieraus, und zwar mit einer Mindestlänge von 12 Nukleotiden, vorzugsweise 30 bis 90 Nukleotiden, im Falle der Nukleinsäuren, und einer Mindestlänge von 4 Aminosäuren, vorzugsweise 10 bis 30 Aminosäuren, im Falle der Peptide oder Proteine.

Der Begriff der differenziellen Bildung des Nm23-H1/GAPDH-Komplexes bezeichnet die höhere, insbesondere um zumindest den Faktor 2 erhöhte Bildung dieses Komplexes in Tumorzellen, verglichen mit normalen Zellen gleichen Gewebes, vorzugsweise gepaart aus einem Patienten.

Der Begriff der Behandlung von Krebs umfasst auch die Behandlung von Metastasen aus Primärtumoren in vom Primärtumor verschiedenen Geweben. Der Begriff der Behandlung umfaßt auch die Prophylaxe.

Die galenische Herrichtung einer erfindungsgemäßen pharmazeutischen Zusammensetzung kann in fachüblicher Weise erfolgen. Als Gegenionen für ionische Verbindungen kommen beispielsweise Na+, K+, Li+ oder Cyclohexylammonium infrage. Geeignete feste oder flüssige galenische Zubereitungsformen sind beispielsweise Granulate, Pulver, Dragees, Tabletten, (Mikro-) Kapseln, Suppositorien, Sirupe, Säfte, Suspensionen, Emulsionen, Tropfen oder injizierbare Lösungen (i.v., i.p., i.m., usw.) sowie Präparate mit protrahierter Wirkstoff-Freigabe, bei deren Herstellung übliche Hilfsmittel wie Trägerstoffe, Spreng-, Binde-, Überzugs-, Quellungs-, Gleit- oder Schmiermittel, Geschmacksstoffe, Süßungsmittel und Lösungsvermittler, Verwendung finden. Als Hilfsstoffe seien Magnesiumcarbonat, Titandioxyd, Lactose, Mannit und andere Zucker, Talcum, Milcheiweiß, Gelatine, Stärke, Zellulose und ihre Derivate, tierische und pflanzliche Öle wie Lebertran, Sonnenblumen-, Erdnuss- oder Sesamöl, Polyethylenglycole und Lösungsmittel, wie etwa steriles Wasser und ein- oder mehrwertige Alkohole, beispielsweise Glycerin, genannt. Eine erfindungsgemäße pharmazeutische Zusammensetzung ist dadurch herstellbar, dass mindestens ein erfindungsgemäß verwendetes Protein oder Peptid in definierter Dosis mit einem pharmazeutisch geeigneten und physiologisch verträglichen Träger und ggf. weiteren geeigneten Wirk-, Zusatz- oder Hilfsstoffen, beispielsweise zytotoxischen Komponenten gemischt und zu der gewünschten Darreichungsform hergerichtet ist.

Zytotoxische Komponenten bzw. Gruppen sind Verbindungen, welche direkt oder indirekt Apoptose einleiten bzw. zu Nekrose führen oder zumindest wachstumshemmend wirken. Es ist möglich, diese mit einem erfindungsgemäßen Peptid oder Protein zu mischen oder daran zu binden. Solche Gruppen bzw. Verbindungen können neben Radioisotopen (z.B. 188Re, 213Bi, 99mTc, 90Y, 131J, 177Lu) insbesondere Zytostatika sein, welche in der Tumortherapie eingesetzt werden. Beispiele hierfür sind: Alkylantien (z.B. Mechlorethamin, Ifosfamid, Chlorambucil, Cyclophosphamid, Melphalan, Alkylsulfonate, Busulphan, Nitrosoharnstoffe, Carmustin, Lomustin, Semustin, Triazene, Dacarbazin), Antimetaboliten (z.B. Folsäure-Antagonisten, Methotrexat, Pyrimidin-Analoga, Fluoruracil, Fluordesoxyuridin, Cytarabin, Gemcitabin, Purin-Analoga, Mercaptopurin), Mitosehemmer (z.B. Vincaalkaloide, Vincristin, Vinblastin, Paclitaxel, Docetaxel, Protaxel), Epipodophyllotoxine (z.B. Etoposid, Teniposid), Antibiotika (z.B. Dactinomycin, Daunorubicin, Idarubicin, Anthracycline, Bleomycin, L-Asparaginase), Platinkomplexverbindungen (z.B. Cisplatin), Hormone und verwandte Verbindungen (z.B. Nebennierenrindensteroide, Aminogluthetimid, Gestagene, Östrogene, Androgene, Antiöstrogene, Tamoxifen, Steriodanaloga, Flutamid). Bei Bindung einer solchen Verbindung mit einem erfindungsgemäßen Peptid oder Protein erfolgt die Kopplung dergestalt, dass die Affinität des Peptids oder Proteins zum Nm23-H1/GAPDH-Komplex um nicht mehr als 90%, vorzugsweise 50%, bezogen auf die Substanz ohne zytostatische Gruppe, reduziert ist und die zytostatische Wirkung der Gruppe um nicht mehr als 90%, vorzugsweise 50%, bezogen auf die Verbindung ohne Substanz, reduziert ist.

Eine immunstimulierende Komponente, welche mit einem erfindungsgemäßen Protein oder Peptid gemischt oder daran gebunden sein kann, ist meist ein Protein oder ein wirksamer Bestandteil hiervon, welches Zellen des Immunsystems stimuliert. Beispiele hierfür sind: Zytokine, wie M-CSF, GM-CSF, G-CSF, Interferone, wie IFN-alpha, -beta, -gamma, Interleukine wie IL-1 bis -16 (außer -8), human LIF, Chemokine wie Rantes, MCAF, MIP-1-alpha, -beta, NAP-1 und IL-8.

Eine Reportergruppe ist ein Atom, Molekül oder eine Verbindung, welche in Verbindung mit einem hierauf abgestellten Assay den Nachweis der Reportergruppe und der somit mit der Reportergruppe verbundenen Verbindung oder Substanz ermöglicht. Beispiele für Reportergruppen und hiermit assoziierte Detektionsmethoden sind: 32P-Labeling und Intensitätsmessung mittels Phosphoimager oder Fluoreszenz. Viele weitere Beispiele sind dem Durchschnittsfachmann bekannt und bedürfen nicht der detaillierten Aufzählung.

Eine an nm23-H1 und/oder GAPDH bindende Substanz kann eine Substanz sein, welche ein nm23-H1 und/oder GAPDH Protein oder eine nm23-H1 und/oder GAPDH RNA bindet.

Im Rahmen der vorstehenden Definition gegenüber dem engen Wortsinn erweiterte Begriffsbestimmungen umfassen auch die bestimmten Begriffe im engen Wortsinn. Ausführungen zu einem Aspekt der Erfindung gelten für andere Aspekte analog.

### Beispiele:

### 1: Methoden.

### 1.1: Expression rekombinanter Proteine und deren Reinigung.

Ein (His)6-nm23-H1/GAPDH Komplex wurde aus einem bicistronischen Baculovirusvektor in Sf9 Insektenzellen exprimiert und durch Affinitätschromatographie an Ni2+-Agarose gereinigt. Hieran schloß sich eine Pseudo-Affinitätschromatographie an Reaktivgelb-Agarose (Sigma) gemäß der Literaturstelle M. Engel et al., J Biol Chem 273:20058-20065 (1998) an. Der komplette kodierende Bereich von PGM B wurde durch reverse Transkriptase PCRamplifiziert (Forward-Primer: 5'-taggatccatggccgcctacaaactg-3', Reverse-Primer: 5'-cgaagcttaatgaaaatgggagccactg-3') und in den pQE30 Expressionsvektor (Qiagen) kloniert. Mit dem erhaltenen Plasmid wurde E.coli JM109 transformiert. Die Bakterien wurden bei 37°C bis zu einem OD600 von 0,5 kultiviert und die Expression durch inkubation mit 0,5 mM IPTG für 3 Stunden induziert. Die Bakterien wurden durch Zentrifugieren geerntet, einer Lyse unterworfen und das rekombinante PGM gereinigt durch Ni2+ Affinitätchromatographie (1. Schritt), und zwar unter gleichen Bedingungen, wie in der vorstehenden Literaturstelle angegeben. In einem zweiten Reinigungsschritt wurde das von der Ni2+-Säule eluierte PGM-Protein über Nacht bei 4°C dialysiert gegen Phosphatpuffer (20 mM K2HPO4/K2HPO4 Puffer, pH 7,1, 0,5 mM DTT). Das Protein wurde dann über eine Hydoxylapatitsäule (Biorad), welche zuvor mit besagtem Phosphatpuffer äquilibriert wurde, laufen gelassen. Gebundenes Protein wurde mit einem linearen Gradienten von 20 mM KH2PO4/K2HPO4 Puffer zu 500 mM KH2PO4/K2HPO4 Puffer eluiert. Die Fraktionen wurden gepoolt, gegen Lagerungspuffer dialysiert (20 mM Tris/HCl, pH 7,5, 40% Glycerol, 40 mM NaCl, 1 mM DTT, 0,5 mM EDTA) und bei -20°C gelagert.
Um einen PGM-H10G Mutanten zu generieren, wurde ortsspezifische Mutagenese mit der Wildtyp-PGM enthaltenden Plasmid durchgeführt, und zwar mit den Mutageneseprimern 5'-GTGCTGATCCGGGGCGGCGAGAGCGCA-3' (vorwärts) und 5'-TGCGCTCTCGCCGCCCCGGATCAGCAC-3' (rückwärts). Im Einzelnen wurde das QuikChange® Kit (Stratagene) entsprechend der Herstellervorschrift verwendet. Expression und Reinigung des Mutanten wurden wie vorstehend für den PGM-Wildtyp beschrieben ausgeführt.

### 1.2: Screening einer cDNA Expressionsbibliothek durch in-situ-Phosphorylierung

Zur Identifizierung von in vitro Substraten des nm23-H1/GAPDH Komplexes wurde eine lambda-TripleEx fetal brain cDNA Expressionsbibliothek (Clontech) gescreent. Die Phagen wurden in LB top Agar enthaltend E.coli Wirtszellen platiert und kultiviert, bis Plaques gerade sichtbar wurden. Phagen (10.000 p.f.u./Platte) wurden für mehrere Stunden auf Nitrozellulosemembranen (Duralose-UV®, Stratagene), welche zuvor in 10 mM IPTG getränkt wurden, geblottet. Die Membranen wurden entfernt und mit 20 mM Tris/HCl, pH 7,0, 150 mM NaCl, 1 mM GTP, 2% BSA, geblockt. Dann erfolgte eine Inkubation bei 30°C mit nm23-H1/GAPDH- Komplex (0,1mg/ml), 15 µM γ32P-GTP (0,37 MBq/ml) in 20 mM Tris/HCl, pH 7,0, 40 mM NaCl, 1 mg/ml BSA in Rollflaschen. Nach dreimaligem Waschen für 5 min. in Puffer (20 mM Tris/HCl, pH 7,0, 150 mM NaCl, 0,1 % NP-40) bei 0°C wurden die Membranen mit dem gleichen Puffer, jedoch zusätzlich enthaltend 1 mM GDP für 10 min. bei 20°C unter Schütteln inkubiert. Die Membranen wurden wiederum, wie vorstehend beschrieben, gewaschen und nass zur Filmbelichtung (Autoradiographie) bei -80°C eingesetzt. Membranen, welche ein Signal erzeugten, wurden mit saurem Puffer (Glycin/HCl, pH 1,5) behandelt und erneut der Autoradiographie unterworfen. Phagen-Plaques, welche mit radioaktiven Spots korrelierten wurden aus der Membran herausgetrennt und für eine zweite Runde des Plattierens und in-situ Screenens auf Phosphorylierung eingesetzt. Nur solche Phagen, die nach der sauren Waschung der ersten Runde ausgesondert waren, produzierten in einer zweiten Runde Signale. Einzelne Phagen-Klone wurden herausgetrennt und die cDNA-Inserts wurden mittels PCR unter Verwendung des "LD-insert screening amplimer set" (Clontech, Kat.Nr. 9107-1) amplifiziert. Die PCR-Produkte wurden in pCR4.1 TOPO Klonierungsvektoren (Invitrogen) subkloniert und die erhaltenen Plasmide sequenziert.

### 1.3: Phosphorylierung von PGM durch den nm23-H1/GAPDH Komplex.

Die Phosphorylierungsassays wurden mit 1 µg rekombinantem PGM und 200 ng nm23-H1/GAPDH Komplex in einem Gesamtvolumen von 25 µl Puffer (20 mM Tris/HCl, pH 7,2, 40 mM NaCl, 5 mM MgCl2, 1 mM DTT) durchgeführt. Die Reaktionen wurden durch Zugabe von γ 32P-GTP (20 µM; 1µCi/Reaktion) gestartet. Nach der Inkubation bei 30°C für 10 min. wurde SDS-Probenpuffer (pH 8,8) zugegeben, und die Proben wurden direkt und ohne vorheriges Erhitzen einer SDS Polyacrylamidgel Elektrophorese aufgetragen. Die SDS-Gele wurden nach Lämmli vorbereitet, mit der Ausnahme, dass für Sammel- und Trenngele Tris/HCl Puffer, pH 8,8, eingesetzt wurde. Nach der Elektrophorese wurden die Gele ohne Fixierung unter Vakuum bei 80 °C getrocknet und über Nacht autoradiographiert.

### 1.4: Peptid-Expression und Reinigung.

Die erfindungsgemäßen Peptide wurden chemisch synthetisiert und auf über 80% Reinheit gereinigt von ThermoHybaid. Alternativ wurden Oligonukleotide, welche für ein erfindungsgemäßes Peptid kodieren (Seq.-ID 4), sowie für ein Kontrollpeptid (MRQIKIWFPNRRMKWKKHHHHHHPWRIEGHL), von ThermoHybaid synthetisiert. Erhaltene 5'-phosphorylierte DNA wurde in die Ndel/EcoRI Schnittstelle eines pET5a Vektors (Stratagene) ligiert. Mit dem Plasmid wurde E.coli BL21pLys transformiert. Nach Kultivierung wurde die Expression durch Inkubation von 0,5 mM IPTG für 2,5 Stunden induziert. Nach Ernte und Lyse der Bakterien erfolgte die Reinigung durch Bindung an Ni2+-Agarose Beads. Nach dem Waschen der Beads erfolgte die Elution der Peptide in einem Puffergradienten sowie eine Dialyse. In den Zellkultur-Experimenten wurden die in PBS/5 M Harnstoff gelösten Peptide durch schnelle Verdünnung in frisches Kulturmedium zugegeben.

### 1.5: Isolierung des Glykolyseenzymkomplexes durch isoelektrische Fokussierung.

Zellen wurden mit einem Homogenisierungspuffer enthaltend 10 mM Tris, 1 mM NaF und 1 mM Mercaptoethanol, pH 7,4, extrahiert. Die isoelektrische Fokussierung erfolgte mit einem linearen Gradienten von Glycerol (50 Vol.-% zu 0 Vol.-%) und Ampholin (pI 3,5 - 10,5), wie in der Literaturstelle S. Mazurek et al., J Cell Physiol 167:238-250 (1996) beschrieben.

### 1.6: Messung der Enzymaktivitäten.

Pyruvatkinase (PK), Nukleosiddiphosphatkinase (nm23-H1) und GAPDH-Aktivitäten wurden gemessen, wie in S. Mazurek et al., J Cell Physiol 167:238-250 (1996) und M. Engel et al., J Bio Chem 273:20058-20065 (1998) beschrieben. Die Messung der PGM Aktivität erfolgte gemäß H.U. Bergmeyer, Methoden der enzymatischen Analyse 3, Band I und II, Verlag Chemie, Weinheim/Bergstraße (1974). Anstelle von 2,3-Bisphosphoglycerat wurde auch 0,5 mM dCTP als Kofaktor eingesetzt. Auch die folgende, andere Methode wurde eingesetzt: PGM-enthaltende Proben wurden in einem Reaktionsmix (30 mM KH2PO4/K2HPO4 Puffer, pH 7,2, 1 mM MgS04, 1,5 mM 2-Phosphoglycerat, 2 mM NAD+, 1 mM DTT, 1 mg/ml BSA, 0,5 mM dCTP und 1 U 3-Phosphoglyceratdehydrogenase in einem Gesamtvolumen von 1 ml) verdünnt. Der Anstieg der UV-Absorption bei 340 nm wurde bestimmt. Die Enzymaktivität wurde in je min. und je ml reduzierte µMol NAD+, units/ml (eNADH = 6,22 cm²/µMol) ausgedrückt.

### 1.8: Zellkultur.

MCF-7- (Brusttumor) und Tx3095- (Glioblastom)-Zelllinien wurden ebenso wie rheumatoide Synoviozyten und menschliche Embryonalzellen in DMEM, supplementiert mit 5 mM Glucose, 2 mM Glutamin, 100 U/ml Penicillin, 100 µg/ml Streptomycin, 10 % FCS und 4 mM Natriumpyruvat, in befeuchteter Luft unter Luft mit 5% CO2 kultiviert. MDA-MB-453 (Brusttumor)-Zellen wurden in Leibovitz L15 Medium kultiviert, welches mit 2 mM Glutamin, 5 mM Natriumpyruvat, 100 U/ml Penicillin, 100 µg/ml Streptomycin, 10 % FCS supplementiert war, bei 37 °C in luftdichten Kulturflaschen gezogen. GLC4-(kleinzelliges Lungenkarzinom)-Zellen wurden kultiviert in RPMI-1640 Medium, supplementiert mit 10% FCS, 5 mM Glucose, 2 mM Glutamin, 100 Units Penicillin/ml, 100 µg Streptomycin/ml, 4 mM Natriumpyruvat. Die Zellen wurden inkubiert in befeuchteter Luft unter Luft mit 5% CO2.

### 1.9: Messung des glycolytischen Flux.

Zellkulturüberstände wurden bei verschiedenen Zelldichten gesammelt und sofort in flüssigem Stickstoff eingefroren. Glucose, Pyruvat, Laktat, Glutamin, Glutamat, Serin und Alanin
wurden in den Überständen gemessen gemäß der Literaturstellen H.U. Bergmeyer, Methoden der enzymatischen Analyse 3, Band I und II, Verlag Chemie, Weinheim/Bergstraße (1974), S. Mazurek et al., J Biol Chem 272:4941-1852 (1997) und F. Hugo et al., J Cell Physiol 153:539-549 (1992). Zur Bestimmung der Fluxraten wurde der Verbrauch oder die Erzeugung des jeweiligen Metaboliten in nMol/(h*Schale) berechnet.

### 1.10 Zelltransfektion.

Je 200.000 Embryonalzellen bzw. Zellen der Kleinzelligen-Lungenkarzinomzellline GLC4 wurden in 6-Well-Zellkulturplatten (Greiner, Deutschland) in dem unter Punkt 1.8 beschriebenen voll supplementiertem Medium ausgesäht. Die Zellen wurden kultiviert unter 95% Luft/5 % CO2 bei 37°C. Nach 16h wurde das Medium entfernt und die Zellen mit einer Mischung aus 5 µl Lipofectin (Invitrogen) und 1 µg Plasmid-DNA in 2 ml Medium, supplementiert wie unter 1.8 beschrieben jedoch ohne FCS, transfiziert. Nach einer zweistündigen Inkubation wurde das Transfektionsmedium wieder durch voll supplementiertes Medium ersetzt. Als Plasmid-DNA wurden entweder der pHOOK-2 mammalian expression vector (Invitrogen) mit der "in frame"-einklonierten kodierenden Sequenz der 2,3-Bisphosphoglyceratmutase-cDNA oder der leere pHOOK-2-Vektor (Kontrolle) verwendet. Da der pHOOK-2-Vektor auch ein β-Galactosidase-Gen enthält, wurde zur Kontrolle auf Erfolg der Transfektionen ein Teil der Zellen mittels X-Gal gefärbt, unter Verwendung des β-gal staining kit (Invitrogen) nach den Angaben des Herstellers.

### 1.11: ELISA für den Nm23-H1/GAPDH-Komplex

Exiqon Protein Immobilizer ®-Platten (Biozym, Oldendorf, Deutschland) wurden über Nacht bei 4°C beschichtet mit einer Lösung von 1,5 ng anti-GAPDH-Antikörper/ml (Rabbit polyclonal von Trevigen (#2275-PC-1) oder Maus monoclonal (ab8245) von Abcam)) in 100 mM NaHCO3-Puffer, pH 9,6. Kontroll-Wells wurden beschichtet mit einer Verdünnung von 1,5ng/ml BSA (Negativkontrolle) und 1ng/ml gereinigte menschliche Nukleosiddiphosphatkinase A (Positivkontrolle) im gleichen Puffer. Kurz vor der Inkubation mit Probenlösungen wurden die Wells 3 mal mit PBS gewaschen. Vorbereitung der Zell- oder Gewebeproben: Tumor- oder zugehöriges Normalgewebe wurde homogenisiert in eiskaltem Lysispuffer bestehend aus 50 mM K2HPO4/KH2PO4, pH 7,4, 10% Glycerin, 1 mM NaF, 1 mM EDTA, 1 mM DTT, Complete ® Protease Inhibitor-Mix (EDTA-frei, Roche Diagnostics) unter Verwendung eines Ultra-Turrax. Das Homogenisat wurde zentrifugiert für 20 min bei 14.000g, 4°C, und der klare Überstand wurde auf Eis bis zur Verwendung aufbewahrt. Mit einem Aliquot davon wurde jeweils eine Bestimmung des Proteingehaltes mit Hilfe des Bradford-Assays (BioRad) durchgeführt. Nur Proben mit mindestens 2 mg/ml Proteingehalt wurden im ELISA eingesetzt. Alle zu vergleichenden Proben wurden durch die Zugabe von eiskaltem Lysispuffer auf gleiche Proteinkonzentrationen eingestellt. Je 100µl der Proben wurden in die ELISA-Platten-Wells pipettiert und darin für eine Stunde bei 4°C unter Schütteln inkubiert. Danach wurden die Probelösungen entfernt und die Wells schnell 3 mal mit eiskaltem Lysispuffer gewaschen. Die Wells wurden noch ein weiteres Mal mit eiskaltem Phosphatpuffer gewaschen (50 mM K2HPO4/KH2PO4, pH 7,4). Danach wurde der Nukleosiddiphosphatkinase(NDPK)-Reaktionsmix (50 mM Tris/HCl (pH 7,5), 50 mM KCl, 10 mM MgCl2, 1 mM ATP, 2 mM dTDP, 1 mM Phosphoenolpyruvat, 10 mM Natriumphosphat, 2 U Pyruvatkinase in die Wells pipettiert (je 100 µl) und dort für 45 min unter Schütteln bei Raumtemperatur belassen. Die Reaktionen wurden gestoppt durch die Zugabe von 150 µl einer 0,1%-Lösung von 2,4-Dinitrophenylhydrazin in 2 N HCl. Der gemischte Inhalt der Wells wurde dann vollständig in 1,5-ml Reaktionsgefäße überführt, sofern keine Deepwell-Platten verwendet wurden. In den Reaktionsgefäßes wurden je 200 µl 10 N NaOH vorgelegt. Nach gründlichem Mischen wurde 1 ml Ethanol zugegeben und erneut gemischt. Das aus dem gebildeten Pyruvat entstandene rot-braune Hydrazon wurde dann photometrisch in Polystyrolküvetten bzw. in einem Plate reader (bei Deepwell-Platten) bei 520 nM Wellenlänge quantifiziert. Die Hintergrundwerte von den BSA-beschichteten Kontrollwells wurden von den Werten der Anti-GAPDH-beschichteten Wells abgezogen.

### 2: Ergebnisse

### 2.1: Identifizierung des Proteinsubstrates, welches vom nm23-H1/GAPDH-Komplex phosphoryliert wird.

Zur Identifizierung des Substrates wurde eine cDNA-Expressionsbibliothek in lambda-Phagen auf Nitrocellulose-Filter transferriert und durch Inkubation mit bakteriell exprimiertem nm23-H1/GAPDH Komplex in der Gegenwart von γ32P-GTP gescreent. Im Einzelnen wurde gemäß dem Methodenteil vorgegangen. Es wurden verschiedene Kandidatenplaques für säurelabile Phophorylierung gefunden. cDNA Inserts selektierter einzelner Phagenklone wurden mittels PCR amplifiziert und sequenziert. Mehrere gefundene cDNA Sequenzen waren dabei von PGM B abgeleitet. Im Ergebnis wurde PGM B als Substrat für den nm23-H1/GAPDH Komplex identifiziert.

### 2.2: Charakterisierung der Phosphorylierung und Identifizierung des phosphorylierten Aminosäurerestes.

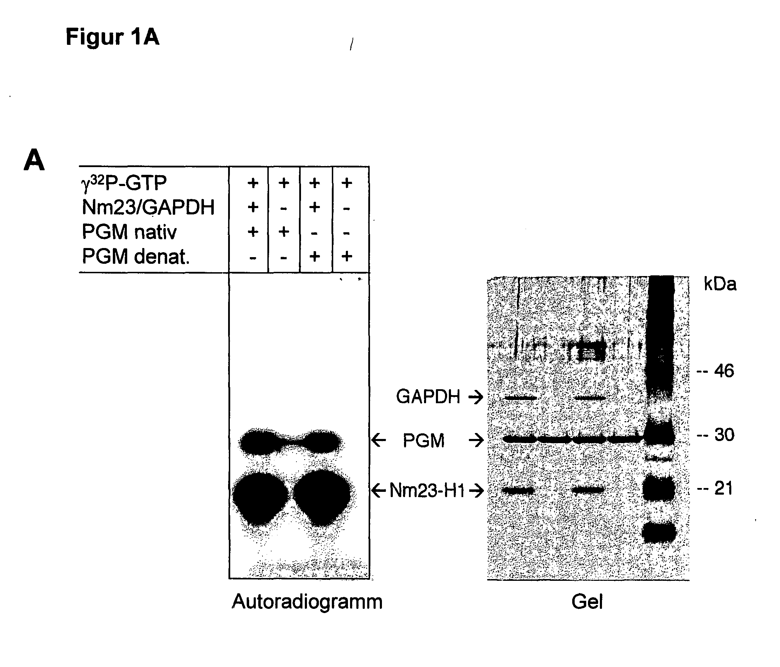

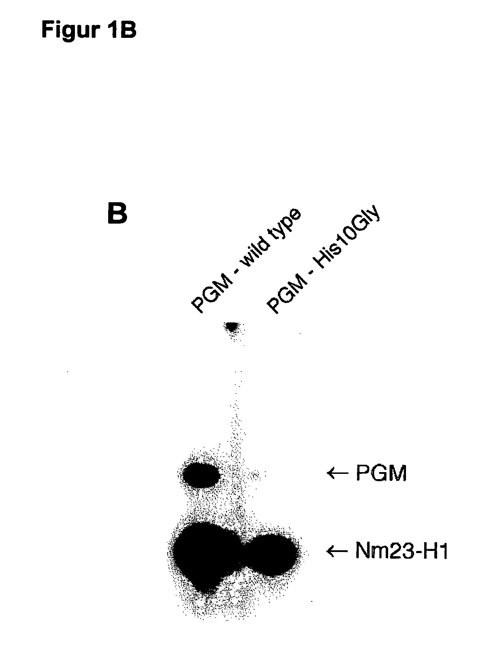

Figur 1A zeigt, dass gereinigtes PGM nur in der Gegenwart des nm23-H1/GAPDH Komplexes und γ 32P-GTP phosphoryliert wird, nicht jedoch in Gegenwart von nur γ 32P-GTP. Die Phosphorylierung findet auch im Falle denaturierter PGM (80 °C für 2 min.) statt, was eventuelle Autophosphorylierungsprozesse ausschließt. In weiteren Versuchen wurde untersucht, ob der H10G Mutant des PGM phosphoryliert wird. Das Ergebnis der Figur 1 B zeigt, dass dies nicht der Fall ist, woraus folgt, dass der Komplex mit diesem Bereich der PGM Sequenz aktivierend wechselwirkt.

### 2.3: PGM-Aktivierung in der Tumorzellinie MCF-7.

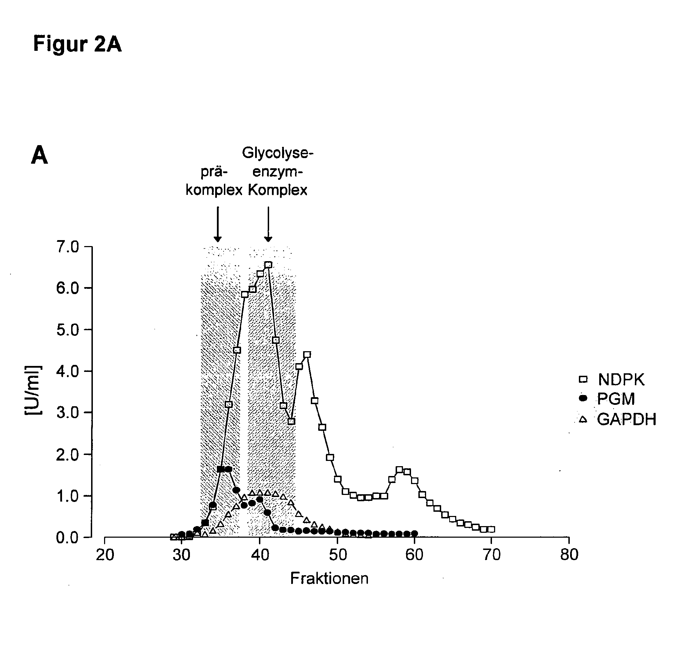

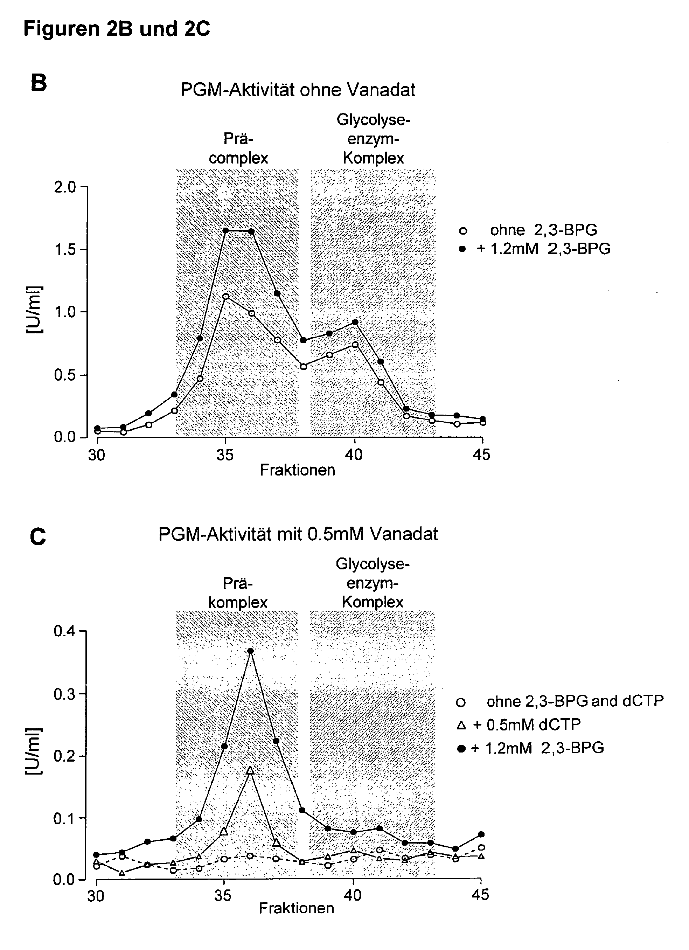

Die Aktivierung von PGM wurde in nativem Glykolyseenzymkomplex der humanen Mammakarzinomzellinie MCF-7 untersucht. Bei der Isoelektrischen Fokussierung von cytosolischem Zellextrakt findet man eine Kofokussierung von PGM mit Nukleosiddiphosphatkinase A (Nm23-H1) und GAPDH im sogenannten Prä-Glykolyseenzymkomplex (Fraktionen 33-37, Figur 2A) und im Glykolyseenzymkomplex (Fraktionen 38-43, Figur 2A). Im Präkomplex wird PGM durch 2,3-Bisphosphoglycerat am deutlichsten aktiviert (Figur 2B). In den gleichen Fraktionen des Präkomplexes findet sich in Gegenwart von 0,5 mM Vanadat neben einer Aktivierung durch 2,3-Bisphosphoglycerat auch eine Aktivierung durch Desoxycytidintriphosphat (dCTP) (Figur 2C). Die Funktion des Vanadats besteht in einer Aktivierung der intrinsischen Phosphataseaktivität der PGM, was dazu führt, dass das aktive Zentrum der PGM einer ständigen Rephosphorylierung bedarf. Dies wird offensichtlich im nativen Komplex durch das dCTP bewirkt. Das Ergebnis belegt, dass in einer bestimmten Untereinheit des Glykolyseenzymkomplexes die Aktivierung der PGM außer durch 2,3-Bisphosphoglycerat auch durch dCTP erfolgen kann, welches ein selektives Substrat der Nukleosiddiphosphatkinase, nicht aber der PGM selbst ist. Dies zeigt indirekt eine Beteiligung der komplexgebundenen Nukleosiddiphosphatkinase an der Aktivierung der PGM.

### 2.4: Effekt eines erfindungsgemäßen Peptids auf die Phosphorylierung von PGM.

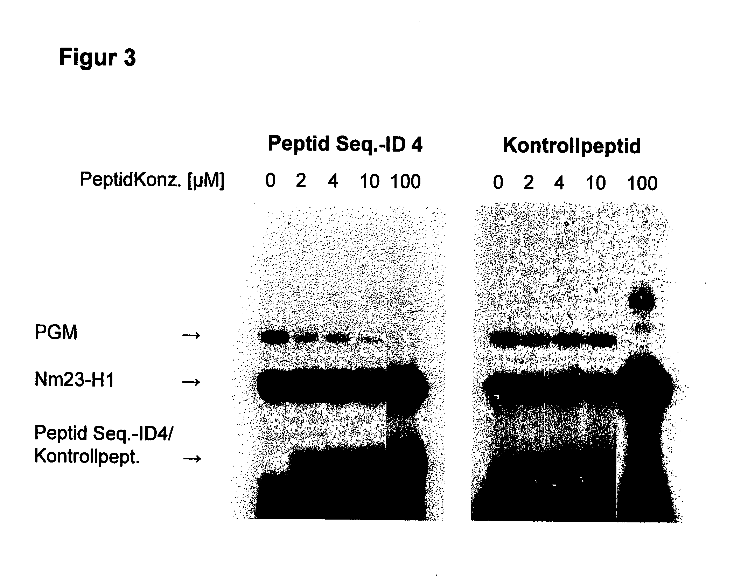

Die vorstehenden Ergebnisse haben gezeigt, dass der nm23-H1/GAPDH-Komplex His 10 der PGM in vitro phosphoryliert, i.e. ein Phosphotransfer von nm23-H1 auf PGM-His 10 erfolgt. Um dies sowie eine Unabhängigkeit von 2,3-Bisphosphoglycerat in intakten Zellen zu überprüfen, wurde das Peptid gemäß Seq.-ID 4 generiert.
In vitro konnte gezeigt werden, dass das Peptid bereits in einer Konzentration von 2 µM die Phosphorylierung von Volllängen-PGM durch den nm23-H1/GAPDH-Komplex effektiv inhibiert, während der Einsatz des Kontrollpeptids keine Inhibierung bewirkte (Figur 3). Das erfindungsgemäße Peptid wurde dabei selbst phosphoryliert, während das Kontrollpeptid selbst bei einer Konzentration von 100 µM nicht phosphoryliert wurde. Das Ergebnis zeigt, dass das erfindungsgemäße Peptid als Substrat des nm23-H1/GAPDH-Komplexes fungiert und dabei die Phosphorylierung der PGM durch den Komplex kompetitiv hemmen kann.

### 2.5: Effekt eines erfindungsgemäßen Peptids auf die Zellproliferation und den Glukosemetabolismus.

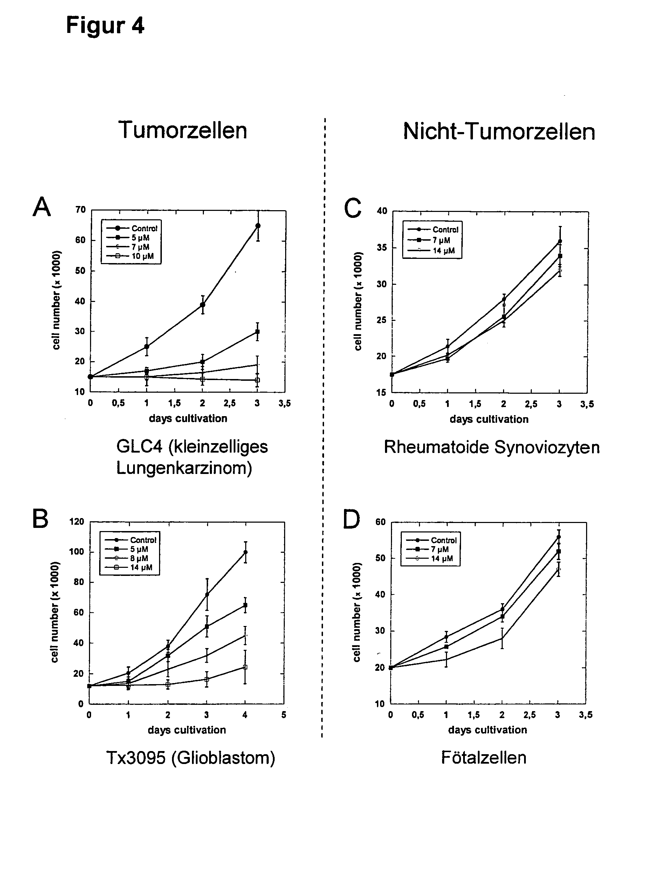

Ein erfindungsgemäßes Peptid wurde auf eine Reihe verschiedener Zelllinien und primärer Zellen angewandt. Die Proliferation von Zellen der Tumorzellinien Tx3095, GLC4, MCF-7 und MDA-MB 453, wurde in einem Konzentrationsbereich von 5 bis 15 µM inhibiert, während der Einsatz des Kontrollpeptids keinen Effekt zeigte. Im Falle von GLC4 wurde Zelltod bereits bei 7 µM festgestellt. Nach Entzug des erfindungsgemäßen Peptids wuchsen die verbleibenden Zellen jedoch normal weiter. Zum Vergleich wurde ein erfindungsgemäßes Peptid bei nicht-tumoralen primären Zellen (Embryonale Zellen, rheumatoide Synoviozyten) getestet. Bei beiden Zelltypen wurde keinerlei Einfluß auf Proliferation oder Lebensfähigkeit beobachtet. Diese erhaltenen Ergebnisse zeigen, dass ein erfindungsgemäßes Peptid tumorzellselektiv funktioniert (Figur 4).

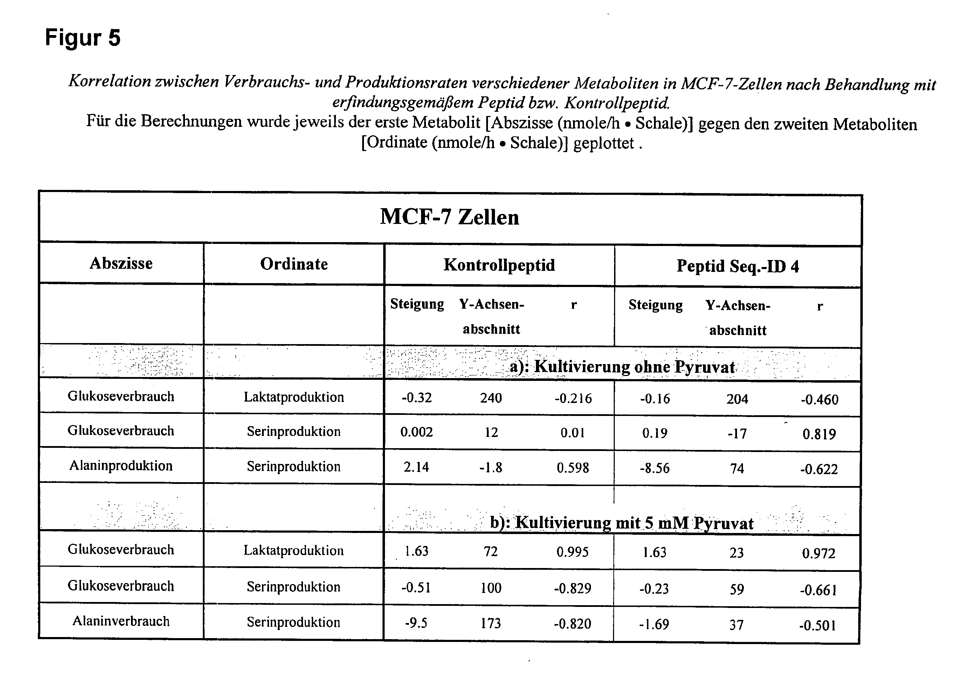

Anhand von der stark glykolytisch aktiven Zellinie MCF7 wurde verifiziert, dass in der Tat der
Glukosemetabolismus mittels eines erfindungsgemäßen Peptids beeinflußt wird. Die Zelllinie wurde mit 7 µM des Peptids (nicht toxische Dosis) behandelt und es wurden die Produktions- bzw. Verbrauchsraten verschiedener Glukosemetaboliten bestimmt. Im Einklang mit der in-vitro beobachteten Hemmung der PGM-Phosphorylierung und -Aktivierung durch den Nm23-H1/GAPDH-Komplex bewirkte das erfindungsgemäße Peptid in den MCF-7-Zellen eine Hemmung der intrazellulären PGM, wie man vor allem an der Änderung der Korrelation zwischen Serinsynthese und Glukoseverbrauch der MCF-7-Zellen erkennen kann. In Gegenwart des erfindungsgemäßen Peptids wird die PGM in der Glykolyseenzymkette gehemmt, wodurch die verbrauchte Glukose fast ausschließlich in Serin umgewandelt wird, was in Gegenwart des Kontrollpeptides nicht der Fall ist. Dies ist auf die Akkumulation des Serin-Vorläufers und PGM-Substrates 3-Phosphoglycerat infolge der PGM-Hemmung durch das erfindungsgemäße Peptid zurückzuführen. Weitere Änderungen in den Metabolitenspiegeln belegen ebenfalls eine Hemmung der PGM durch das erfindungsgemäße Peptid (Figur 5).
Die weiteren Ergebnisse mit MCF-7-Zellen, welche mit zusätzlicher Zugabe von Pyruvat erhalten wurden, nämlich Kompensation des reduzierten Flux von Glucose zu Laktat, belegen, dass ein erfindungsgemäßes Peptid ausschließlich inhibierend auf die Glykolyse, i.e. selektiv auf PGM, und nicht etwa auch (oder stattdessen) auf die Glutaminolyse oder ganz andere Mechanismen wirkt.

Zusammenfassend ist die selektive Hemmung der Proliferation von Tumorzellinien auf die PGM-inhibierende Wirkung des erfindungsgemäßen Peptids und die damit verbundene Störung des Tumorzellen-spezifischen Glukosemetabolismus zurückzuführen.

### 2.6: Überexpression von nm23-H1/GAPDH Komplex in Tumorzellen

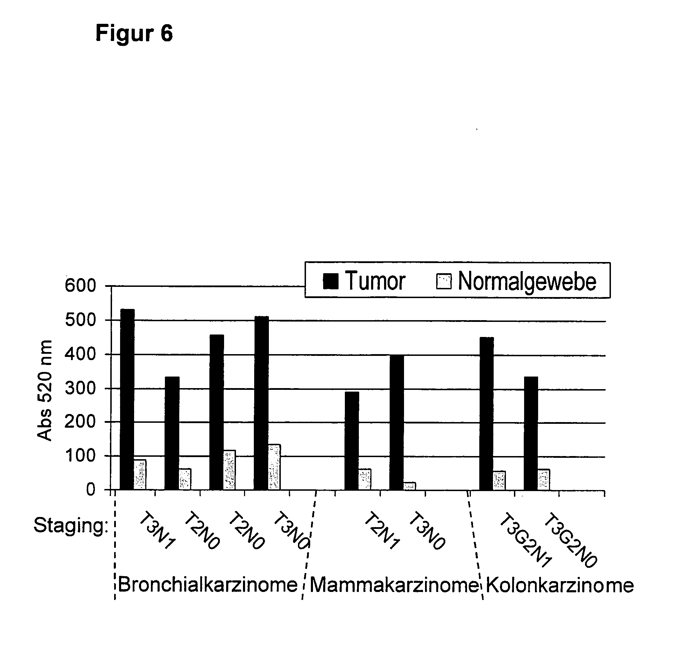

In der Figur 6 sind die Ergebnisse eines ELISA für den nm23-H1/GAPDH Komplex in verschiedenen gepaarten Gewebeproben (Tumorgewebe/Normalgewebe) jeweils aus einem Patienten und für verschiedene Tumorarten dargestellt. Man erkennt in allen Fällen einen erheblich höheren Komplexpegel im Tumorgewebe, verglichen mit den Normalgewebe des gleichen Patienten.

Dies belegt, dass der nm23-H1/GAPDH Komplex ein sehr effektives Target für Screeningverfahren ist, mittels welcher sowohl diagnostische als auch therapeutische Substanzen, insbesondere synthetische chemische Moleküle mit niedrigem Molekulargewicht, identifizierbar sind. Im Falle des Screenens nach therapeutischen Substanzen sind diese entweder Inhibitoren des Komplexes selbst (i.e. seiner Bildung), ermittelbar durch beispielsweise eine Testung auf Reduktion der Komplexpegel in Tumorgewebe insbesondere mit einem ELISA gemäß Figur 6, oder kompetitiv mit PGM an den Komplex bindende Substanzen, wodurch die PGM-Aktivierung inhibiert wird.

### 2.7 Transfektion von Embryonalzellen und GLC4-Zellen mit Vektoren enthaltend die kodierende Sequenz der menschlichen 2,3-Bisphosphoglyceratmutase

Bereits 24 h nach der Transfektion der Kleinzelligen-Lungenkarzinomzellline GLC4 waren über 80% der mit dem 2,3-Bisphosphoglyceratmutase-enthaltenden Plasmid transfizierten Zellen abgestorben (Figur 7). Im Gegensatz dazu zeigten sich keine toten Zellen nach Transfektion mit dem gleichen Plasmid das keine 2,3-Bisphosphoglyceratmutase-Sequenz enthielt.
Mit menschlichen Embryonalzellen wurden nach Durchführung der Transfektionen unter gleichen Bedingungen weder mit dem 2,3-Bisphosphoglyceratmutase-enthaltenden pHOOK-2-Plasmid noch mit dem leeren pHOOK-2-Plasmid tote Zellen gefunden. Die mit dem 2,3-Bisphosphoglyceratmutase-enthaltenden Plasmid transfizierten Zellen zeigten lediglich eine 40%ige Redution der Zellteilungsrate. Die Transfektionsrate lag bei beiden Zelltypen unter den verwendeten Bedingungen bei etwa 30%, wenn nach der X-Gal-Färbung auch die schwach blau gefärbten Zellen als positiv gewertet wurden.
Dieses Ergebnis zeigt, dass die artifizielle Expression der 2,3-Bisphosphoglyceratmutase in der Tumorzellinie GLC4 zum Zelltod führt. Da dieses Enzym in gesunden, normalen Zellen ohnehin exprimiert wird, stellt die Einschleusung eines Vektors zur transienten Überexpression der 2,3-Bisphosphoglyceratmutase eine vielverprechende Option zur Therapie von malignen Tumorerkrankungen dar.

## Patentansprüche

1. Verwendung von PGM oder von einem PGM-enthaltendem Zelllysat, oder von mit PGM angereicherten cytoplasmatischen Fraktionen in einem Screeningverfahren zur Findung von die PGM-Aktivität inhibierenden Substanzen, **dadurch gekennzeichnet dass** eine prospektive Substanz oder eine Mischung prospektiver Substanzen mit PGM, dem Zelllysat oder der Fraktion kontaktiert wird, wobei in einem Assay die PGM-Aktivität bestimmt wird (vorzugsweise mit und ohne prospektive Substanzen), und wobei eine die PGM-Aktivität reduzierende Substanz oder Mischung von Substanzen selektiert wird.

2. Methode nach Anspruch 1, wobei die selektierte Substanz oder die Mischung von Substanzen nochmals mit PGM, dem Zelllysat oder der Fraktion kontaktiert wird, jetzt jedoch in Gegenwart von 2,3-Bisphosphoglycerat und/oder 1,3- Bisphosphoglycerat, wobei in einem Assay die PGM-Aktivität bestimmt wird, wobei solche Substanzen selektiert werden, deren hemmende Wirkung auf die PGM-Aktivität durch die Anwesenheit von 2,3-Bisphosphoglycerat und/oder 1,3-Bisphosphoglycerat zumindest teilweise aufgehoben wird.

3. Methode nach Anspruch 1 oder 2, **dadurch gekennzeichnet, dass** Orthovanadat zugegeben wird.

4. Methode nach Anspruch 1, 2 oder 3, **dadurch gekennzeichnet, dass** ein Nukleosidtriphosphat oder ein Gemisch von mehreren Nukleosidtriphosphaten zugegeben wird, auswählbar aus der Gruppe "Adenosintriphosphat, Guanosintriphosphat, Cytidintriphosphat, Uridintriphosphat, Thymidintriphosphat, Inosintriphosphat, Xanthosintriphosphat, Desoxyadenosintriphosphat, Desoxyguanosintriphosphat, Desoxycytidintriphosphat, Desoxyuridintriphosphat, Desoxythymidintriphosphat, Desoxyinosintriphosphat, Desoxyxanthosintriphosphat, Didesoxyadenosintriphosphat, Didesoxyguanosintriphosphat, Didesoxycytidintriphosphat, Didesoxyuridintriphosphat, Didesoxythymidintriphosphat, Didesoxyinosintriphosphat , Didesoxyxanthosintriphosphat".

5. Verwendung von BisPGM oder von BisPGM-enthaltendem Zelllysat oder von mit BisPGM angereicherten cytoplasmatischen Fraktionen zur Findung von die BisPGM-Aktivität steigernden Substanzen, wobei in einem Assay die BisPGM-Aktivität bestimmt wird.

6. Methode zur Findung von die Transkriptions- oder Translationsrate der BisPGM erhöhenden Substanzen, **dadurch gekennzeichnet dass** ein Nukleinsäurevektor, enthaltend die BisPGM-Genpromotorsequenz und/oder die 3 '-nichttranslatierte Sequenz der BisPGMmRNA in Kombination mit einem exprimierbaren Reportergen in Zellen eingebracht werden, wonach die Zellen enthaltend den besagten Nukleinsäurevektor mit den prospektiven Substanzen in Kontakt gebracht werden, wobei die Aktivität des Reportergens gemessen wird, wobei die Substanzen selektiert werden, welche die Aktivität des Reportergenproduktes erhöhen.

7. Peptid mit der Aminosäurensequenz gemäß Seq.-ID 1 oder Seq.-ID 4.

8. Peptid mit der Aminosäurensequenz gemäß Seq.-ID 1, wobei die Aminogruppe oder C-terminale Carboxylgruppe der Seq.-ID 1 mit einem Transportmolekül verbunden ist, welches vorzugsweise ausgewählt ist aus der Gruppe bestehend aus "Seq.-ID 2, Lectine, synthetische Polymere, Zucker, Riboflavin, Antennapedia-Homöodomäne-Peptid von Drosophila, HIV-tat-Peptid, EnterotoxinB-Protein von E.coli, Importpeptid-Carrier, Arginin-Oligomere, Herpes Simplex Virus Typ 1 (HSV-1) VP22-Polypeptid".

9. Peptid gemäß Anspruch 8, wobei zwischen der Aminosäurensequenz gemäß Seq.-ID 1 und dem Transportmolekül eine Aminosäuresequenz gemäß Seq.-ID 3 oder eine Teilsequenz hieraus mit zumindest 4 aufeinanderfolgenden Aminosäuren eingebaut ist, oder wobei besagte Sequenz gemäß Seq.-ID 3 oder besagte Teilsequenz hieraus mit der freien N-terminalen Aminogruppe oder der freien C-terminalen Carboxylgruppe von Seq.-ID 1 verbunden ist.

10. Nukleinsäure codierend für ein Protein oder Peptid gemäß einem der Ansprüche 7 bis 9.

11. Vektor enthaltend eine Nukleinsäure nach Anspruch 10.

12. Zelle, welche mit einem Vektor gemäß Anspruch 11 transformiert ist.

13. Methode zur Quantifizierung der Proteinphosphorylierungs-Aktivität des Nm23-H1/GAPDH-Komplexes oder der PGM-phosphorylierenden Enzymaktivität, **dadurch gekennzeichnet dass** ein Peptid oder Protein gemäß einem der Ansprüche 7 bis 9, vorzugsweise versehen mit einem Affinitätstag, ausgewählt aus der Gruppe "(His)6, Strep-Tag, Biotin, Gluthathion-S-Transferase, Maltose-bindendes Protein, Calmodulin" mit einem Testlysat aus tumorverdächtigen Zellen oder mit rekombinantem Nm23-H1/GAPDH-Komplex kontaktiert wird, wobei das Peptid oder Protein als Substrat der endogenen PGM-phosphorylierenden Enzymaktivität oder des Nm23-H1/GAPDH-Komplexes entweder durch ein zugegebenes γ32P-Nukleosidtriphosphat radioaktiv phosphoryliert wird und der Einbau ggf. nach Binden des Peptids an eine feste Matrix und Waschen quantifiziert wird, oder die Menge an phosphoryliertem Peptid oder Protein nach Einbau von nicht-radioaktivem Phosphat durch Binden des Peptids an beispielsweise ein Eisenchelat bestimmt wird, wobei das Peptid oder Protein mit einer Reportergruppe versehen bzw. mit einer Reportersubstanz gekoppelt ist.

14. Verwendung von Nm23-H1 und/oder GAPDH oder einer Teilsequenz hieraus oder Nm23-H1/GAPDH-Komplex zum Screenen nach kompetitiv mit PGM daran bindenden Substanzen oder nach die Bildung des Nm23-H1/GAPDH-Komplexes inhibierenden oder inaktivierenden Substanzen, oder zum Screenen nach prospektiven Detektorsubstanzen, **dadurch gekennzeichnet dass** eine prospektive Substanz oder eine Mischung solcher prospektiver Substanzen mit besagtem Protein oder Peptid oder Komplex kontaktiert wird, wobei mit einem Bindungsassay Bindungsereignisse detektiert werden oder die Proteinphosphorylierungs-Aktivität des Nm23-H1/GAPDH-Komplexes gemäß Anspruch 13 bestimmt wird, und wobei eine bindende, inhibierende oder inaktivierende prospektive Substanz, ggf. nach Dekonvolutierung, selektiert wird.

15. Verwendung einer nach Anspruch 14 identifizierten, an den Nm23-H1/GAPDH-Komplex bindenden Detektorsubstanz zur Detektion von Krebs, und/oder von Metastasen aus Primärtumoren, oder zur Detektion eines Risikos der Erkrankung an Krebs, wobei eine Gewebeprobe in vitro oder in vivo auf Expression oder Überexpression des Nm23-H1/GAPDH-Proteinkomplexes untersucht wird, ggf. standardisiert gegen eine Normalgewebeprobe des gleichen oder eines verschiedenen Patienten, wobei die Detektorsubstanz vorzugsweise eine Reportergruppe enthält, wobei Bindung von Nm23-H1 und/oder GAPDH an die Detektorsubstanz halbquantitativ oder quantitativ detektiert wird.

16. Verfahren nach Anspruch 15, **dadurch gekennzeichnet, dass** eine Detektorsubstanz in einer Ausführungsform mit einer Reportergruppe in zu untersuchendes Gewebe (in vivo oder in vitro) appliziert wird, wobei das zu untersuchende Gewebe dann einer Detektionsverfahrenstufe unterworfen wird, welche sensitiv für die Reportergruppe ist, und wobei im Fall der Detektion eines definierten Mindestwertes der Reportergruppe im Gewebe das Gewebe als Tumorzellen enthaltend qualifiziert wird.

17. Methode zur Quantifizierung des Nm23-H1/GAPDH-Komplexes, **dadurch gekennzeichnet dass** eine GAPDH- oder Nm23-H1-bindende Substanz auf einer festen Matrix immobilisiert wird, diese Matrix dann mit der GAPDH/Nm23-H1-Komplex-enthaltenden Probe in Kontakt gebracht wird, wobei die besagte Matrix nach dem In-Kontakt-Bringen durch Waschen von ungebundenem Material befreit wird, wonach die Menge an denjenigem Komplexbindungspartner, der nicht unmittelbar an die auf der besagten Matrix immobilisierten Substanz gebunden ist, durch einen enzymatischen Assay oder durch Bindung einer mit einer Reportergruppe versehenen Detektorsubstanz quamtifiziert wird.

18. Verwendung von BisPGM oder eines katalytisch aktiven Teilstückes daraus, wobei das BisBGM-Protein oder Teilstück daraus kovalent oder nicht-kovalent mit einem Transportmolekül verbunden ist, welches vorzugsweise ausgewählt ist aus der Gruppe bestehend aus "Seq.-ID 2, Lectine, synthetische Polymere, Zucker, Riboflavin, Antennapedia-Homöodomäne-Peptid von Drosophila, HIV-tat-Peptid, EnterotoxinB-Protein von E.coli, Importpeptid-Carrier, Arginin-Oligomere, Herpes Simplex Virus Typ 1 (HSV-1) VP22-Polypeptid, Chariot®-Reagenz", zur Herstellung einer pharmazeutischen Zusammensetzung.

19. Methode zur Erhöhung des Gehaltes an 2,3-BPG in den Zellen eines Organismus, **dadurch gekennzeichnet dass** eine pharmazeutische Zusammensetzung enthaltend eine Verbindung selektiert nach Anspruch 5 und/oder 6 verabreicht wird.

20. Methode zur Erhöhung des Gehaltes an 2,3-BPG in den Zellen eines Organismus, **dadurch gekennzeichnet dass** eine Nukleinsäure in die Zellen des Organismus eingeschleust wird, welche in einem exprimierbaren Abschnitt die komplette kodierende Sequenz der BisPGM, oder eine katalytisch aktive Teilsequenz daraus, und wahlweise zusätzlich im Leserahmen anfusioniert eine Transporterpeptidsequenz enthält, vorzugsweise ausgewählt aus der Gruppe "Seq-ID 2, Antennapedia-Homöodomäne-Peptid von Drosophila, HIV-tat-Peptid, EnterotoxinB-Protein von E.coli, Importpeptid-Carrier, Arginin-Oligomere, Herpes Simplex Virus Typ 1 (HSV-1) VP22-Polypeptid".

21. Methode zur Erhöhung des Gehaltes an 2,3-BPG in den Zellen eines Organismus, **dadurch gekennzeichnet dass** eine pharmazeutische Zusammensetzung gemäß Anspruch 18 verabreicht wird.

22. Verwendung einer der Methoden nach Anspruch 19, 20 oder 21 zur Behandlung von Krebs, insbesondere von bösartigen Neoplasien und Metastasen.

23. Verwendung eines Proteins oder Peptids nach einem der Ansprüche 7 bis 9 zur Herstellung einer pharmazeutischen Zusammensetzung.

24. Verwendung von 2,3-Bisphosphoglycerat, 2-Phosphoglycerat, 3-Phosphoglycerat, 1,3-Bisphosphoglycerat, 2,3-Bis(sulfoamino)propionsäure, 2,3-Bis(phosphonothio)propionsäure, 2,3-Bisphosphonopropionsäure, 2-Carboxybutan-1,4-bisphosphonsäure, 1,2-Bis(sulfoamino)-1-(1*H*-tetrazol-5-yl)ethan oder zyklischem 2,4-Diphosphoglycerat, oder einer Kombination von mindestens zwei dieser Verbindungen zur Herstellung einer pharmazeutischen Zusammensetzung.

25. Pharmazeutische Zusammensetzung, insbesondere zur Behandlung von Krebs, bevorzugt von bösartigen Neoplasien und Metastasen., enthaltend einen Wirkstoff nach einem der Ansprüche 7 bis 9, 18, 23 oder 24 in physiologisch wirksamer Dosis, vorzugsweise in galenischer Herrichtung zur i.v., i.p., subkutanen oder intraläsionalen Injektion.
